# EUROPEAN PATENT SPECIFICATION

(11) **EP 2 083 819 B1**
(45) Date of publication and mention of the grant of the patent: **22.02.2017**
(21) Application number: 07845135.8
(22) Date of filing: 21.11.2007
(51) Int. Cl.: A61K 31/404, A61K 31/4184, A61K 31/4402, A61K 31/47, A61K 31/4965, A61K 31/517, A61K 31/542, C07D 215/12, C07D 235/08, C07D 235/10, A61P 25/18

(54) **PDE10 INHIBITORS AND RELATED COMPOSITIONS AND METHODS**
PDE10-HEMMER UND VERWANDTE ZUSAMMENSETZUNGEN UND VERFAHREN
INHIBITEURS DE LA PDE10 ET COMPOSITIONS ET PROCÉDÉS APPARENTÉS

(30) Priority: 21.11.2006 US 866745 P; 22.03.2007 US 896386 P
(43) Date of publication of application: 05.08.2009
(73) Proprietor: Omeros Corporation, Seattle, WA 98119 (US)
(72) Inventor: CUTSHALL, Neil S., Snohomish, WA 98290 (US); JOHNSTON, Derek, Glasgow Scotland G69 8LA (GB); UROGDI, Lazlo, 1095 Budapest (HU); BERGMANN, John E., Mercer Island, WA 98040 (US); ONRUST, Rene, Mercer Island, WA 98040 (US); HONGKUI, Zeng, Shoreline, WA 98133 (US); CSEH, Sandor, 2120 Dunakeszi (HU); PAPP, Akos, 1078 Budapest (HU)
(74) Representative: Grünecker Patent- und Rechtsanwälte PartG mbB
(86) International application number: PCT/US2007/085440
(87) International publication number: WO 2008/064342

(56) References cited:
- WO-A-00/73280
- WO-A-02/32896
- WO-A-2005/037779
- WO-A-2005/070419
- WO-A-2006/033318
- WO-A-2006/113236
- WO-A-2006/127396
- WO-A-2007/008529
- WO-A-2008/020920
- US-A1- 2003 018 047
- DAYAM R ET AL: "Diketo acid pharmacophore. 2. Discovery of structurally diverse inhibitors of HIV-1 integrase" JOURNAL OF MEDICINAL CHEMISTRY, AMERICAN CHEMICAL SOCIETY. WASHINGTON, vol. 48, no. 25, 15 December 2005 (2005-12-15), pages 8009-8015, XP002412213 ISSN: 0022-2623
- DATABASE REGISTRY Chemical Library, Supplier: Sigma-Aldrich 11 May 2006 (2006-05-11), XP002488205 retrieved from STN accession no. RN 883805-25-2
- DATABASE REGISTRY Chemical Library, Supplier: Sigma-Aldrich 11 May 2006 (2006-05-11), XP002488206 retrieved from STN accession no. RN 883826-67-3

## Description

This invention relates generally to compounds having activity as PDE10 inhibitors, and to compositions containing the same, as well as to compounds for use in treating schizophrenia by administration of such compounds to a warm-blooded animal in need thereof.

Cyclic nucleotide phosphodiesterases (PDEs) are represented by a large superfamily of enzymes. PDEs are known to possess a modular architecture, with a conserved catalytic domain proximal to the carboxyl terminus, and regulatory domains or motifs often near the amino terminus. The PDE superfamily currently includes more than twenty different genes subgrouped into eleven PDE families (Lugnier, C., "Cyclic nucleotide phosphodiesterase (PDE) superfamily: a new target for the development of specific therapeutic agents." Pharmacol Ther. 2006 Mar; 109(3):366-98).

A recently described PDE, PDE10, was reported simultaneously by three independent groups (Fujishige et al., "Cloning and characterization of a novel human phosphodiesterase that hydrolyzes both cAMP and cGMP (PDE10A)," J Biol Chem 1999, 274:18438-18445; Loughney et al., "Isolation and characterization of PDE10A, a novel human 3', 5'-cyclic nucleotide phosphodiesterase," Gene 1999, 234:109-117; Soderling et al., "Isolation and characterization of a dual-substrate phosphodiesterase gene family: PDE10A," Proc Natl Acad Sci USA 1999, 96:7071-7076). PDE10 has the capacity to hydrolyze both cAMP and cGMP; however, the *K*ₘ for cAMP is approximately 0.05 µM, whereas the *K_{M}* for cGMP is 3 µM. In addition, the *V*ₘₐₓ for cAMP hydrolysis is fivefold lower than for cGMP. Because of these kinetics, cGMP hydrolysis by PDE10 is potently inhibited by cAMP *in vitro,* suggesting that PDE10 may function as a cAMP-inhibited cGMP phosphodiesterase *in vivo.* Unlike PDE8 or PDE9, PDE10 is inhibited by IBMX with an IC₅₀ (50% inhibitory concentration) of 2.6 µM. *(See* Soderling and Beavo, "Regulation of cAMP and cGMP signaling: new phosphodiesterases and new functions," Current Opinion in Cell Biology, 2000, 12:174-179.)

PDE10 contains two amino-terminal domains that are similar to the cGMP-binding domains of PDE2, PDE5 and PDE6, which are domains conserved across a wide variety of proteins. Because of the wide conservation of this domain, it is now referred to as the GAF domain (for the GAF proteins: cGMP binding phosphodiesterases; the cynobacterial *Anabaena* adenylyl cyclase; and the *Escherichia coli* transcriptional regulator fhlA). Although in PDE2, PDE5 and PDE6 the GAF domains bind cGMP, this is probably not the primary function of this domain in all cases (*e.g., E. coli* are not thought to synthesize cGMP). Interestingly, *in vitro* binding studies of PDE10 indicate the dissociation constant (K_{d}) for cGMP binding is well above 9 µM. As *in vivo* concentrations of cGMP are not thought to reach such high levels in most cells, it seems likely that either the affinity of PDE10 for cGMP is increased by regulation, or that the primary function of the GAF domain in PDE10 may be for something other than cGMP binding.

Inhibitors of the PDE family of enzymes have widely been sought for a broad indication of therapeutic uses. Reported therapeutic uses of PDE inhibitors include allergies, obtrusive lung disease, hypertension, renal carcinoma, angina, congestive heart failure, depression and erectile dysfunction (WO 01/41807 A2). Other inhibitors of PDE have been disclosed for treatment of ischemic heart conditions (U.S. Pat. No. 5,693,652). More specifically, inhibitors of PDE10 have been disclosed for treatment of certain neurological and psychiatric disorders including, Parkinson's disease, Huntington's disease, schizophrenia, delusional disorders, drug-induced psychosis and panic and obsessive-compulsive disorders (U.S. Patent Application No. 2003/0032579). PDE10 has been shown to be present at high levels in neurons in areas of the brain that are closely associated with many neurological and psychiatric disorders. By inhibiting PDE10 activity, levels of cAMP and cGMP are increased within neurons, and the ability of these neurons to function properly is thereby improved. Thus, inhibition of PDE10 is believed to be useful in the treatment of a wide variety of conditions or disorders that would benefit from increasing levels of cAMP and cGMP within neurons, including those neurological, psychotic, anxiety and/or movement disorders mentioned above.

WO2006/113236 (A2) discloses a method of treating an inflammatory disorder in a subject. The method involves administering to a subject an effective amount of a compound that modulates soluble adenylyl cyclase, thereby treating the inflammatory disorder in the subject. WO2006/113236 (A2) also relates to a method of inhibiting respiratory burst in adherent neutrophils without inhibiting neutrophil degranulation in or bacterial killing by neutrophils. The method involves contacting adherent neutrophils with an effective amount of a compound that modulates soluble adenylyl cyclase.

WO2005/070419 (A1) discloses a method of treating a disorder mediated by soluble adenylyl cyclase in a subject. The method involves administering to a subject an effective amount of a compound disclosed herein that modulates soluble adenylyl cyclase, under conditions effective to treat the disorder mediated by soluble adenylyl cyclase. WO2005/070419 (A1) also relates to a method of treating a disorder mediated by soluble adenylyl cyclase in a subject, where the disorder is selected from the group consisting of learning or memory disorders, malaria, fungal infection, spinal cord injury, Alzheimer's disease, amyotrophic lateral sclerosis, and peripheral neuropathy. The method involves modulating soluble adenylyl cyclase in the subject. Another aspect relates to a method of modulating soluble adenylyl cyclase. The method involves contacting eukaryotic cells with a compound that modulates soluble adenylyl cyclase, under conditions effective to modulate soluble adenylyl cyclase.

WO2005/037779 (A2) discloses medicinally used substances which specifically inhibit peptidases splitting Gly-Pro-p-nitroanilide. WO2005/037779 (A2) further relates to the use of at least one such substance or at least one pharmaceutical or cosmetic composition containing at least one such substance for preventing and treating diseases, particularly diseases with an overshooting immune response (autoimmune diseases, allergies, and transplant rejections), other chronic inflammatory diseases, neuronal diseases, brain damages, skin diseases (acne and psoriasis, among others), tumor diseases, and special viral infections (including SARS).

WO02/32896 (A1) discloses pharmaceutical compositions comprising 1-(furazanyl-3-yl)-[1,2,3]triazole derivatives for the treatment and/or prevention of disorders and diseases, wherein an inhibition of GSK-3 (glycogen synthase kinase-3) is beneficial, especially Alzheimer's disease, bipolar disorder, IGT (impaired glucose tolerance), Type 1 diabetes, Type 2 diabetes and obesity.

WO2006/033318 (A1) discloses a compound useful as an NMDA antagonist having a wide safety region which is a therapeutic agent for Alzheimer's disease, vascular dementia (vascular agnosia), Parkinson's disease, ischemic apoplexy, and pains or a preventive agent for these. The compound is an amine derivative or salt thereof characterized by comprising an amine-containing structure (A) and a di- or tricyclic fused ring (indane, tetralone, 4,5,6,7-tetrahydrobenzothiophene, 4,5,6,7-tetrahydrobenzofuran, 7,8-dihydro-6H-indeno[4,5-b]furan, 2,3-dihydro-1H-cyclopenta[a]naphthalene, etc.) bonded to the structure (A) through e.g. a bond or lower alkylene. The NMDA antagonist contains the derivative or salt as an active ingredient.

WO2006/127396 (A1) relates to compounds, their uses for the elucidation of PAR2 activity and their uses for the treatment or prevention of diseases or disorders related to PAR2 activity, wherein the compound is a derivative of 2-oxo-4-phenyl-3-pyrrolidinecarboxylic acid.

WO2008/020920 (A1) discloses dioxaborolan derivatives such as isonicotinic acid [2-(4,4,5,5-tetramethyl-[1,3,2]dioxaborolan-2-yl)benzylidene]-hydrazide along with compositions containing the same and methods of use thereof in treating oxidative stress.

Dayam R. et al. discloses the discovery of structurally diverse inhibitors of HIV-1 integrase in the Journal of medicinal chemistery, American Chemical Society, Washington, vol. 48, no. 25, pages 8009 - 8015.

WO2007/008529 (A2) discloses compounds and methods useful as inhibitors of cholesterol absorption for the treatment or prevention of cholesterol-related diseases, such as atherosclerosis.

WO00/73280 (A1) discloses novel cathecol hydrazone derivatives.

US2003/018047 (A1) discloses a method for treating certain neurologic and psychiatric disorders in mammals, including humans, comprising administration of a selective PDE10 inhbitor. In particular, US2003018047 (A1) relates to treatment of mood, movement, and anxiety disorders; psychosis; drug, for example alcohol, addiction; disorders having as a symptom deficient cognition; and neurodegenerative disorders and conditions. US2003/018047 (A1) furthermore provides the use of papaverine as a selective inhibitor of PDE10. US2003/018047 (A1) also provides assays for identifying chemical compounds that have activity as selective PDE10 inhibitors.

While advances have been made with regard to inhibition of PDE10, there remains a need in the field for inhibitors of PDE10, as well as the need to treat various conditions and/or disorders that would benefit from the same.

The present invention provides a compound for use in treating schizophrenia in a warm-blooded animal in need thereof, said compound having the following structure (I): 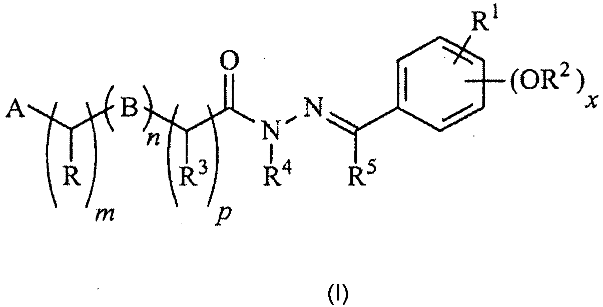 or a pharmaceutically acceptable salt, stereoisomer, or solvate thereof,
wherein:
m, *n* and p are individually 0 or 1;
x is 1, 2 or 3;
A is an optionally substituted heterocycle;
B is -O-, -NR⁶- or -S(O)_{z}- where z is 0, 1 or 2;
R is hydrogen or oxo;
R¹ is absent or represents 1, 2 or 3 substituents that are the same or different and independently halogen, C₁₋₆alkyl, -CHF₂, -CF₃, -CH₂NH₂, -CH₂NH(C₁₋₆alkyl) or -CH₂N(C₁₋₆alkyl)₂;
R² is at each occurrence the same or different and independently hydrogen, C₁₋₆alkyl, -C(=O)(C₁₋₆alkyl), benzyl, -CH₂CONH₂, -CHF₂, -CF₃, or any two R² groups may be taken together to form a C₁₋₆alkanediyl; and
R³, R⁴, R⁵ and R⁶ are the same or different and independently hydrogen or C₁₋₆alkyl.

In another embodiment, the present invention provides the use of a compound for the preparation of a medicament for use in treating schizophrenia in a warm-blooded animal in need thereof, said compound having the following structure (I): 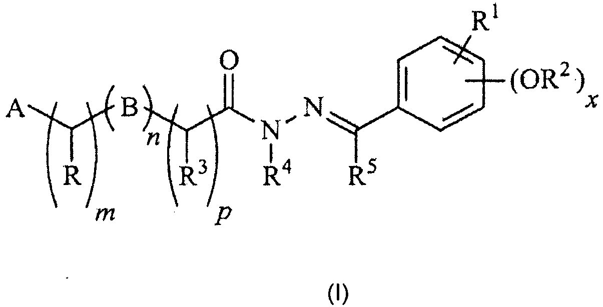 or a pharmaceutically acceptable salt, stereoisomer, or solvate thereof, wherein:
m, *n* and p are individually 0 or 1;
x is 1, 2 or 3;
A is an optionally substituted heterocycle;
B is -O-, -NR⁶- or -S(O)_{z}- where z is 0, 1 or 2;
R is hydrogen or oxo;
R¹ is absent or represents 1, 2 or 3 substituents that are the same or different and independently halogen, C₁₋₆alkyl, -CHF₂, -CF₃, -CH₂NH₂, -CH₂NH(C₁₋₆alkyl) or -CH₂N(C₁₋₆alkyl)₂;
R² is at each occurrence the same or different and independently hydrogen, C₁₋₆alkyl, -C(=O)(C₁₋₆alkyl), benzyl, -CH₂CONH₂, -CHF₂, -CF₃, or any two R² groups may be taken together to form a C₁₋₆alkanediyl; and
R³, R⁴, R⁵ and R⁶ are the same or different and independently hydrogen or C₁₋₆alkyl.

In another embodiment, the present invention provides a compound having the following structure (IVg): 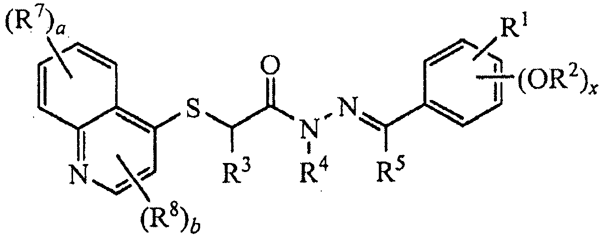 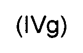 or a pharmaceutically acceptable salt, stereoisomer, or solvate thereof, wherein:
a is 1, 2, 3 or 4;
b is 1 or 2;
x is 1, 2 or 3 with the proviso that when x is 1, R¹ is not a single methyl substituent;
R¹ represents 1 or 2 substitutents independently halogen, C₁₋₆alkyl, -CHF₂ or -CF₃;
R² is at each occurrence the same or different and independently, hydrogen, C₁₋₆alkyl, benzyl, -CH₂CONH₂, -CHF₂, -CF₃, or any two R² groups may be taken together to form a C₁₋₆alkanediyl; and
R³, R⁴ and R⁵ are the same or different and independently hydrogen or C₁₋₆alkyl; and
R⁷ and R⁸ are the same or different and independently hydrogen, halogen, C₁₋₆alkyl, -O(C₁₋₆alkyl), C₁₋₆haloalkyl or nitro.

In another embodiment, the present invention provides a pharmaceutical composition comprising a compound of the invention and a pharmaceutically acceptable carrier or diluent.

Preferred embodiments are set forth in the subclaims.

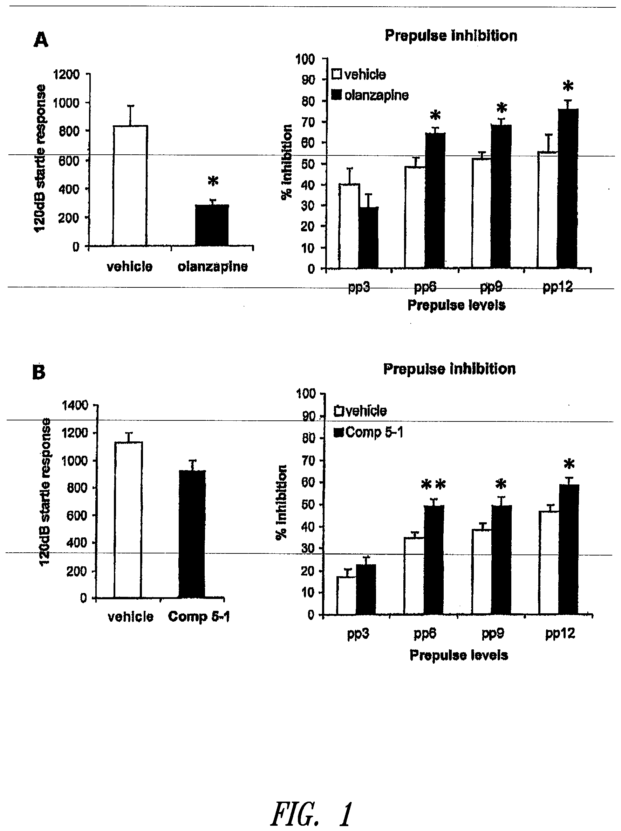

FIGURE 1 illustrates that compound 5-1 (Table 3) increases prepulse inhibition (PPI) similar to olanzapine. C57BL/6 male mice were injected intraperitoneally (i.p.) with either compound or vehicle as described in Example 9. FIGURE 1A shows that olanzapine (3 mg/kg) significantly decreases the startle response (left panel) and increases PPI at 3 different prepulse levels (right panel) compared to vehicle control (* p<0.05, n=8 per group, student's t-test). FIGURE 1B shows that compound 5-1 (50 mg/kg) does not affect the startle response (left panel) but significantly increases PPI at 3 different prepulse levels (right panel) compared to vehicle control (* p<0.05, ** p<0.01, n=24 per group, student's t-test).

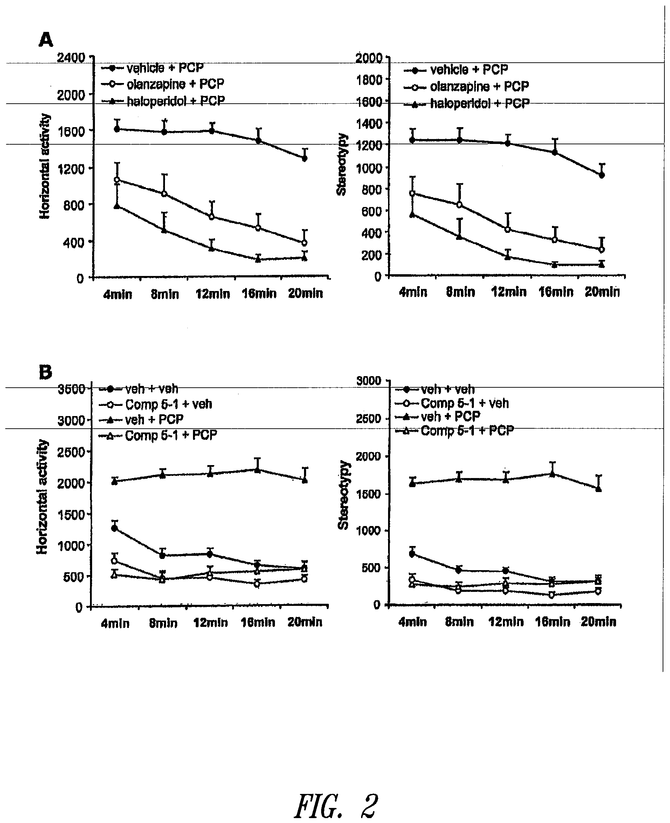

FIGURE 2 illustrates that compound 5-1 (Table 3) reduces PCP-induced hyperactivity similar to olanzapine and haloperidol as described in Example 10. FIGURE 2A shows that both olanzapine (0.2 mg/kg) and haloperidol (0.2 mg/kg) significantly reduce the hyperactivity (left panel) and stereotypy (right panel) induced by PCP as seen in the vehicle+PCP control (p<0.001, n=8 per group, repeated measures ANOVA). FIGURE 2B shows that compound 5-1 (50 mg/kg) completely abolishes the hyperactivity (left panel) and stereotypy (right panel) induced by PCP as seen in the vehicle+PCP control (p<0.001, n=8 per group, repeated measures ANOVA).

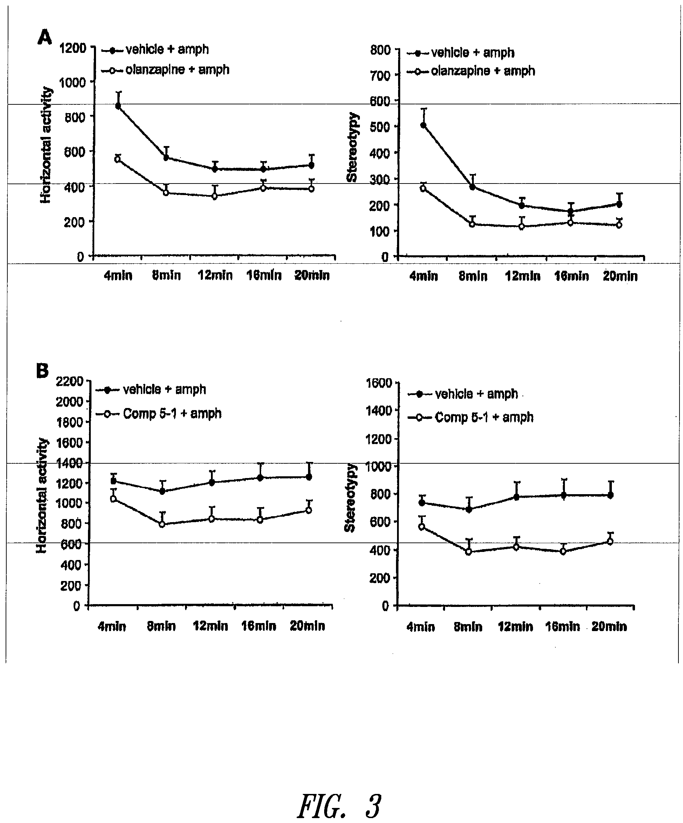

FIGURE 3 illustrates that compound 5-1 (Table 3) reduces amphetamine-induced hyperactivity similar to olanzapine, as described in Example 11. FIGURE 3A shows that olanzapine (0.2 mg/kg) partially but significantly reduce the hyperactivity (left panel) and stereotypy (right panel) induced by amphetamine ("amph") as seen in the vehicle+amph control (p<0.05, n=8 per group, repeatedmeasures ANOVA). FIGURE 3B shows that compound 5-1 (50 mg/kg) also partially but significantly reduce the hyperactivity (left panel) and stereotypy (right panel) induced by amphetamine as seen in the vehicle+amph control (p<0.01, n=8 per group, repeated measures ANOVA).

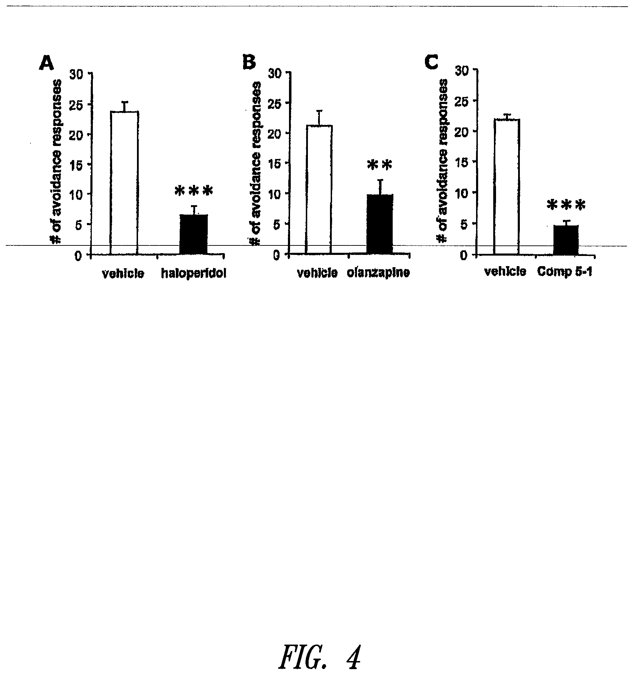

FIGURE 4 illustrates that compound 5-1 (Table 3) reduces Conditioned Avoidance Response (CAR) similar to haloperidol and olanzapine, as described in Example 12. FIGURE 4A shows that haloperidol (0.15 mg/kg) significantly reduces the number of avoidance response (*** p<0.001, n=6 per group, paired t-test). FIGURE 4B shows that olanzapine (0.45 mg/kg) significantly reduces the number of avoidance response (** p<0.01, n=6 per group, paired t-test). FIGURE 4C shows that compound 5-1 (30 mg/kg) significantly reduces the number of avoidance response (*** p<0.001, n=6 per group, paired t-test). In all these cases, the numbers of escape response increase correspondingly and the total numbers of transitions between the two chambers do not change (data not shown), indicating a specific reduction of CAR that is not due to compromised motor function.

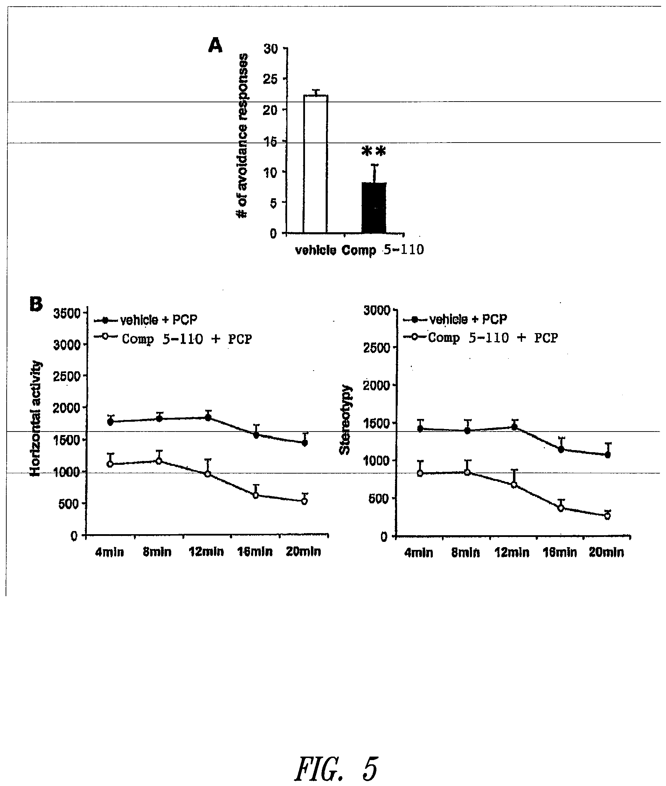

FIGURE 5 illustrates that compound 5-110 (Table 3) exhibits antipsychotic properties in two behavioral tests, as described in Example 13. FIGURE 5A shows that compound 5-110 (10 mg/kg) significantly reduces the number of avoidance response in the CAR test (** p<0.01, n=6 per group, paired t-test). FIGURE 5B shows that compound 5-110 (30 mg/kg) significantly reduces the hyperactivity (left panel) and stereotypy (right panel) induced by PCP (5 mg/kg) (p<0.001, n=8 per group, repeated measures ANOVA).

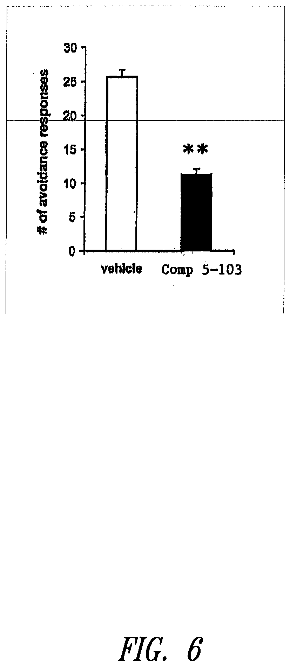

FIGURE 6 illustrates that compound 5-103 (Table 3) reduces Conditioned Avoidance Response (CAR), as described in Example 14. Compound 5-103 (10 mg/kg) significantly reduces the number of avoidance response (** p<0.01, n=6 per group, paired t-test).

As used herein, the above terms have the following meaning:
"C₁₋₆alkyl" means a straight chain or branched, noncyclic or cyclic, unsaturated or saturated aliphatic hydrocarbon containing from 1 to 6 carbon atoms. Representative saturated straight chain alkyls include methyl, ethyl, n-propyl, n-butyl, n-pentyl, n-hexyl, and the like; while saturated branched alkyls include isopropyl, *sec-*butyl, isobutyl, *tert*-butyl*,* isopentyl, and the like. Representative saturated cyclic alkyls include cyclopropyl, cyclobutyl, cyclopentyl, cyclohexyl, and the like; while unsaturated cyclic alkyls include cyclopentenyl and cyclohexenyl, and the like. Unsaturated alkyls contain at least one double or triple bond between adjacent carbon atoms (referred to as an "alkenyl" or "alkynyl", respectively). Representative straight chain and branched alkenyls include ethylenyl, propylenyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like; while representative straight chain and branched alkynyls include acetylenyl, propynyl, 1-butynyl, 2- butynyl, 1--pentynyl, 2-pentynyl, 3-methyl-1-butynyl, and the like.

"C₁₋₆alkanediyl" means a divalent C₁₋₆alkyl from which two hydrogen atoms are taken from the same carbon atom or from different carbon atoms, such as-CH₂-, -CH₂CH₂-, -CH=CH-, -CH=C(CH₃)-, -CH₂CH₂CH₂-, -CH₂CH=CH-,-CH(CH₃)CH₂CH₂-, -CH₂C(CH₃)₂CH₂-, and the like.

"Heterocycle" means a 4- to 7-membered monocyclic, or 7- to 10-membered bicyclic, heterocyclic ring which is either saturated, unsaturated or aromatic, and which contains from 1 to 4 heteroatoms independently selected from nitrogen, oxygen and sulfur, and wherein the nitrogen and sulfur heteroatoms may be optionally oxidized, and the nitrogen heteroatom may be optionally quaternized, including bicyclic rings in which any of the above heterocycles are fused to a benzene ring. The heterocycle may be attached via any heteroatom or carbon atom. An aromatic heterocycle is referred to herein as a "heteroaryl", and includes furyl, benzofuranyl, thiophenyl, benzothiophenyl, pyrrolyl, indolyl, isoindolyl, azaindolyl, pyridyl, quinolinyl, isoquinolinyl, oxazolyl, isooxazolyl, benzoxazolyl, pyrazolyl, imidazolyl, benzimidazolyl, thiazolyl, benzothiazolyl, isothiazolyl, pyridazinyl, pyrimidinyl, pyrazinyl, triazinyl, cinnolinyl, phthalazinyl, oxadiazolyl, thiadiazolyl, benzisoxazolyl, triazolyl, tetrazolyl, indazolyl and quinazolinyl. In addition to the heteroaryls listed above, heterocycles also include morpholinyl, pyrrolidinonyl, pyrrolidinyl, piperidinyl, piperazinyl, and the like.

The term "optionally substituted" as used in the context of an optionally substituted heterocycle (as well heteroaryl) means that at least one hydrogen atom is replaced with a substituent. In the case of an oxo substituent ("=O") two hydrogen atoms are replaced. When substituted, one or more of the above groups are substituted. "substituents" within the context of this invention include halogen, hydroxy, oxo, cyano, nitro, amino, alkylamino, dialkylamino, alkyl, alkoxy, alkylthio, haloalkyl, aryl, arylalkyl, heteroaryl, heteroarylalkyl, heterocycle and heterocyclealkyl, as well as-NRₐR_{b}, -NRₐC(=O)R_{b}, -NRₐC(=O)NRₐNR_{b}, -NRₐC(=O)OR_{b} -NRₐSO₂R_{b}, -C(=O)Rₐ,-C(=O)ORₐ, -C(=O)NRₐR_{b}, -OC(=O)NRₐR_{b}, -ORₐ, -SRₐ, -SORₐ, -S(=O)₂Rₐ, -OS(=O)₂Rₐ and -S(=O)₂ORₐ. In addition, the above substituents may be further substituted with one or more of the above substituents, such that the substituent is a substituted alkyl, substituted aryl, substituted arylalkyl, substituted heterocycle or substituted heterocyclealkyl. Rₐ and R_{b} in this context may be the same or different and independently hydrogen, alkyl, haloalkyl, substituted alkyl, aryl, substituted aryl, arylalkyl, substituted arylalkyl, heterocycle, substituted heterocycle, heterocyclealkyl or substituted heterocyclealkyl.

In one embodiment, both *m* and *n* are 0 and *p* is 1, and the compounds have the following structure (II) or a pharmaceutically acceptable salt, stereoisomer, or solvate thereof:

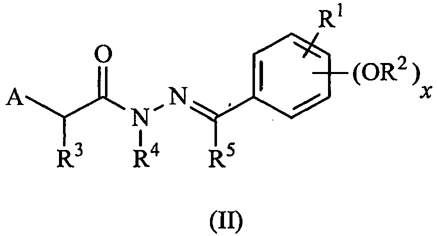

In another embodiment, *m, n* and *p* are 1, and the compounds have the following structure (III) or a pharmaceutically acceptable salt, stereoisomer, or solvate thereof:

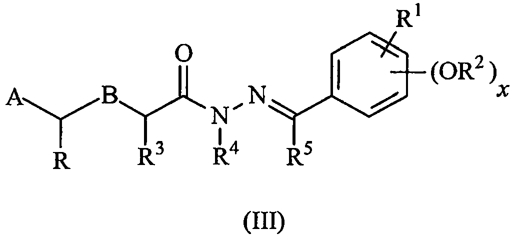

In another embodiment, *m* is 0 and *n* and *p* are both 1, and the compounds have the following structure (IV) or a pharmaceutically acceptable salt, stereoisomer, or solvate thereof:

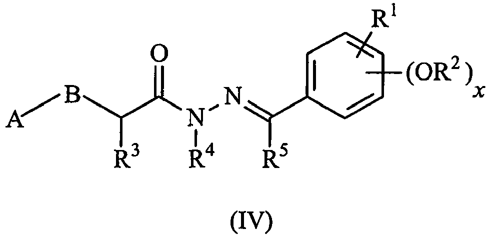

In another embodiment, both *m* and *p* are 1 and *n* is 0, and the compounds have the following structure (V) or a pharmaceutically acceptable salt, stereoisomer, or solvate thereof:

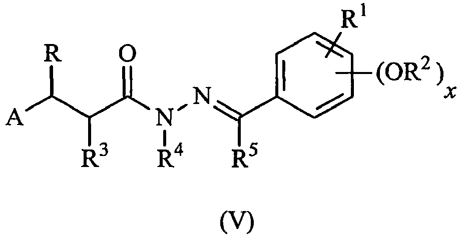

In another embodiment, each of *m, n* and p are 0, and the compounds have the following structure (VI) or a pharmaceutically acceptable salt, stereoisomer, or solvate thereof:

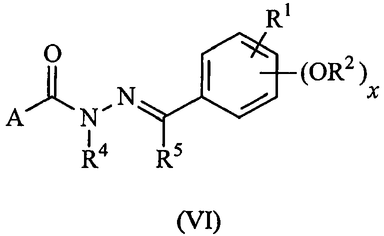

In more specific embodiments of structure (II), R³, R⁴ and R⁵ are hydrogen.

In more specific embodiments of structure (II), R² is methyl or difluoromethyl.

In even more specific embodiments of structure (II), R¹ is absent and x is 3 with the R² groups at the 3, 4 and 5 positions, as represented by structure (IIa):

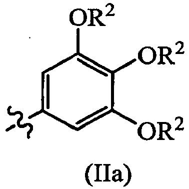

In yet further specific embodiments of structure (II), A is 1*H*-indol-3-yl or 2*H-*benzo[*b*][1,4]thiazin-3(4*H*)-one-2-yl.

In more specific embodiments of structure (IV), B is -S-, -O- or -NR⁶-, respectively, and compounds have the following structures (IVa), (IVb) and (IVc):

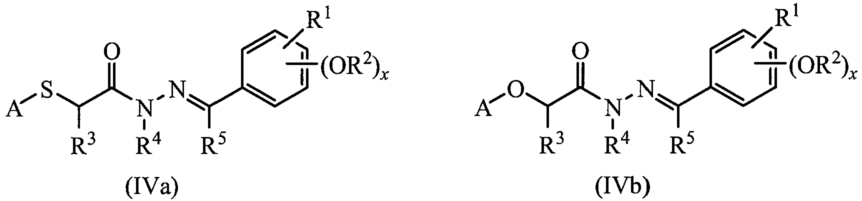

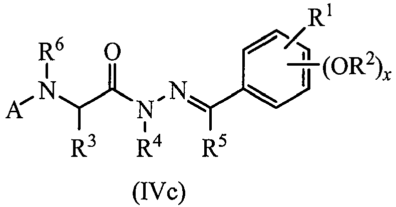

In more specific embodiments of structures (IVa), (IVb) and (IVc), R³, R⁴, R⁵ and R⁶ are hydrogen.

In more specific embodiments of structures (IVa), (IVb) and (IVc), R² is 5 hydrogen, methyl, ethyl, difluoromethyl, 2-propeneyl, 2-methylpropyl, C₁alkanediyl, -CH₂CONH₂ and more specifically methyl.

In more specific embodiments of structures (IVa), (IVb) and (IVc), R¹ is absent or halogen, x is 2 with the R² groups located at the 3 and 4 positions, or x is 3 with the R² groups located at the 3, 4 and 5 positions, as represented by structures (IVd) 10 and (IVe), respectively:

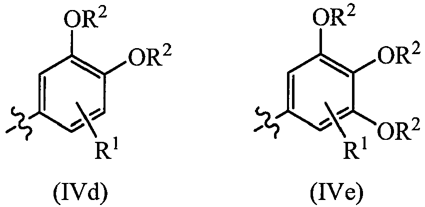

In more specific embodiments of structures (IVa), (IVb) and (IVc), R¹ is 15 2 substituents that are the same or different and independently halogen, such as fluorine, as represented by structure (IVf):

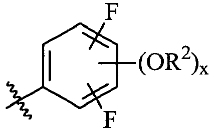

In yet further specific embodiments of structure (IVa), A is an optionally substituted quinolin-4-yl as represented by structure (IVg) where *a* is 1, 2, 3 or 4, *b* is 1 or 2 and R⁷ and R⁸ are the same or different and independently hydrogen, halogen, C₁₋₆alkyl, -O(C₁₋₆alkyl), C₁₋₆haloalkyl or nitro.

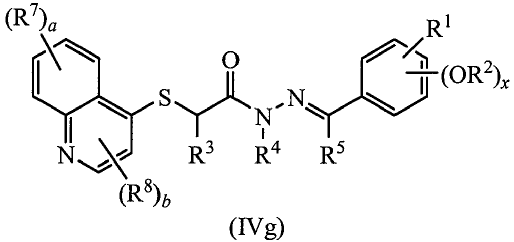

In yet further specific embodiments of structure (IVg), *x* is 1 with the proviso that R¹ is not hydrogen or methyl.

In yet further specific embodiments of structure (IVg), *x* is 2 or 3 with the proviso that R¹ is not hydrogen.

In yet further specific embodiments of structure (IVg), A is 2-methylquinolyn-4-yl, quinolin-4-yl, 2-methyl-6-nitroquinolin-4-yl, 6-bromoquinolin-4-yl, 8-methoxy-5-methylquinolin-4-yl, 6,7-dimethoxy-2-methylquniolin-4-yl, 8-methoxyquinolin-4-yl, 7-(trifluoromethyl)quinolin-4-yl, 2-methyl-8-(trifluoromethyl)quinolin-4-yl or 6-methoxyquinolin-4-yl.

In yet further specific embodiments of structure (IVa), A is an optionally substituted 1-benzyl-1*H*-benzo[*d*]imidazol-2-yl as represented by structure (IVh) where c is 1, 2, 3, 4 or 5 and R⁹ is hydrogen or halogen.

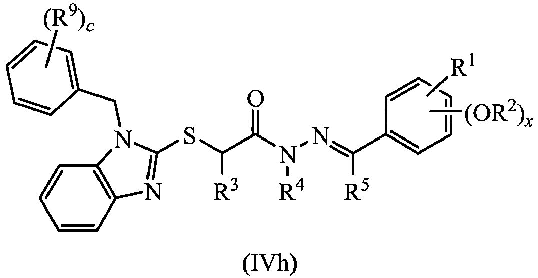

In yet further specific embodiments of structure (IVh), A is 1-benzyl-1*H*-benzo[*d*]imidazol-2-yl, 1-(2-chlorobenzyl)-1*H-*benzo[*d*]imidazol-2-yl or 1-(4-chlorobenzyl)-1*H-*benzo[*d*]imidazol-2-yl.

In more specific embodiments of structures (I) through (VI) above, any two two R² groups, or any R² group and R¹ group, may be taken together to form a C₁₋₆alkanediyl. For example, when two R² groups are taken together to form a C₁₋₆alkanediyl (such as -CH₂-) representative compounds have the following structure (I-1), and when an R² group and R¹ group are taken together to form a C₁₋₆alkanediyl (such as -CH=C(CH₃)-) representative compounds have the following structure (I-2):

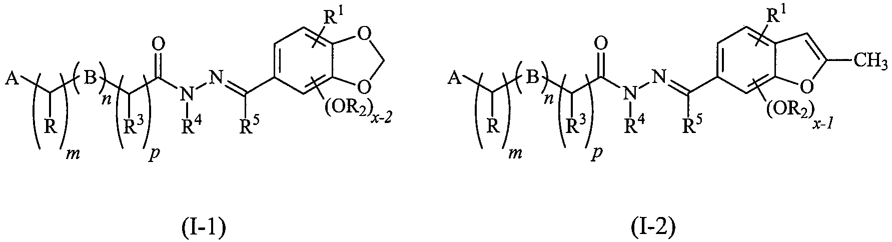

The compounds of the present invention may be prepared by known organic synthesis techniques, including the methods described in more detail in the Examples, or in some instances may be obtained from commercially available sources. In general, the compounds of structure (I) above may be made by the following reaction schemes, wherein all substituents are as defined above unless indicated otherwise.

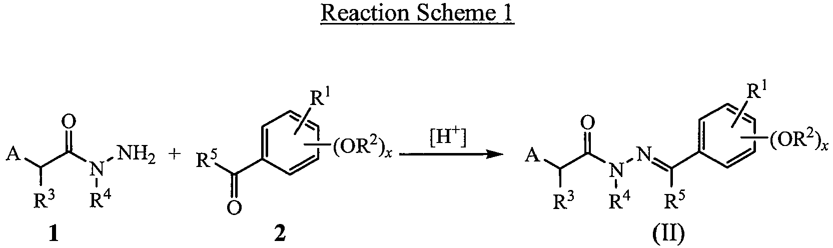

Compounds of formula (1) can be obtained commercially or synthesized through standard literature methods. Compounds of formula (1) can be reacted with a variety of benzaldehyde derivatives of formula (2) in alcoholic solvents, such as ethanol, and in the presence of a catalytic amount of acid, such as acetic acid, and under reflux to provide compounds of structure (II).

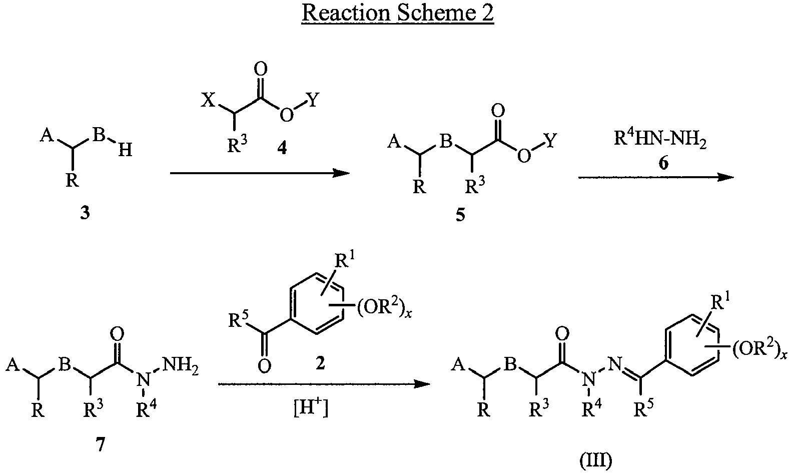

Compounds of formula (3) can be prepared by standard methods or purchased commercially and reacted with compounds of formula (4) where X=Br, Cl and the like, and Y=Me, Et and the like in an appropriate alcoholic solvent such as ethanol and in the presence of a base, such as potassium carbonate and heated to reflux to provide compounds of formula (5). Compounds of formula (5) in ethanol can then be reacted with a hydrazine compound, such as (6), at reflux to provide compounds of formula (7). Compounds of formula (7) can then be reacted with a variety of aryl carbonyl derivatives of formula (2) in alcoholic solvents, such as ethanol, and in the presence of a catalytic amount of acid, such as acetic acid, and under reflux to provide compounds of structure (III).

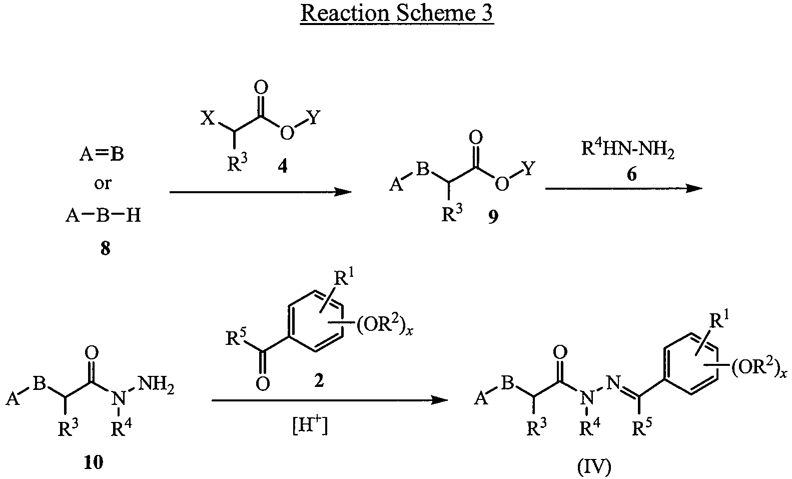

Compounds of formula (8) can be purchased commercially or prepared via standard methods. As shown in Scheme 3, compounds of formula (8) can be reacted with compounds of formula (4) where X=Br, Cl and the like and Y=Me, Et and the like in an appropriate alcoholic solvent, such as ethanol, and in the presence of a base, such as potassium carbonate, and heated to reflux to provide compounds of formula (9). Compounds of formula (9) in ethanol can then be reacted with a hydrazine compound, such as (6), at reflux to provide compounds of formula (10). Compounds of formula (10) can then be reacted with a variety of aryl carbonyl derivatives of formula (2) in alcoholic solvents, such as ethanol, and in the presence of a catalytic amount of acid, such as acetic acid, and under reflux to provide compounds of structure (IV).

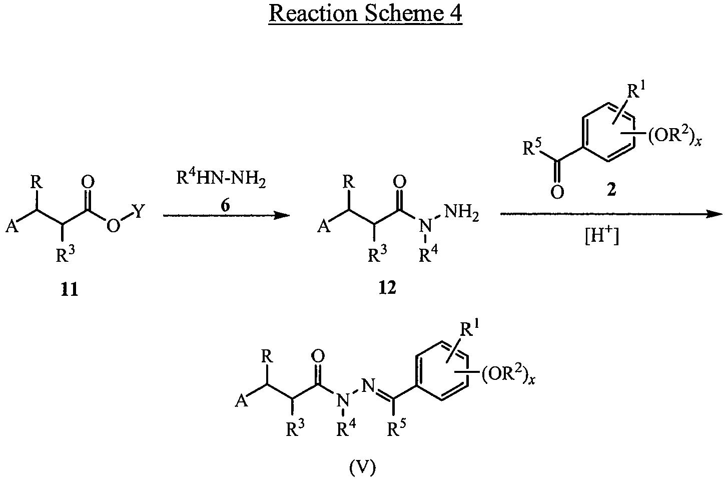

Compounds of formula (11) where Y=Me, Et, and the like can be obtained commercially or prepared via standard methods. Compounds of formula (11) in ethanol can be reacted with a hydrazine compound such as (6) to obtain compounds of formula (12). Compounds of formula (12) can then be reacted with a variety of benzaldehyde derivatives, such as (2), in alcoholic solvents, such as ethanol, and in the presence of a catalytic amount of acid, such as acetic acid, and under reflux to provide compounds of structure (V).

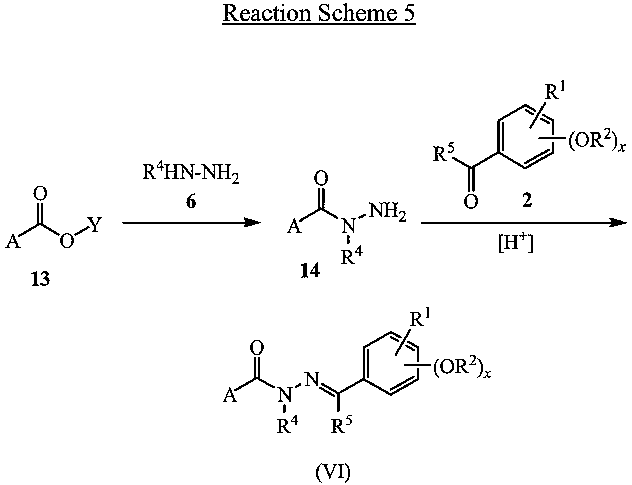

Compounds of formula (13) where Y=methyl (Me),ethyl (Et), etc. can be obtained commercially or prepared via standard methods. Compounds of formula (13) in ethanol can be reacted with a hydrazine compound, such as (6), to obtain compounds of formula (14). Compounds of formula (14) can then be reacted with a variety of benzaldehyde derivatives, such as (2), in alcoholic solvents, such as ethanol, and in the presence of a catalytic amount of acid, such as acetic acid, and under reflux to provide compounds of structure (VI).

The compounds of the present invention may generally be utilized as the free acid or free base. Alternatively, the compounds of this invention may be used in the form of acid or base addition salts. Acid addition salts of the free amino compounds of the present invention may be prepared by methods well known in the art, and may be formed from organic and inorganic acids. Suitable organic acids include maleic, fumaric, benzoic, ascorbic, succinic, methanesulfonic, acetic, trifluoroacetic, oxalic, propionic, tartaric, salicylic, citric, gluconic, lactic, mandelic, cinnamic, aspartic, stearic, palmitic, glycolic, glutamic, and benzenesulfonic acids. Suitable inorganic acids include hydrochloric, hydrobromic, sulfuric, phosphoric, and nitric acids. Base addition salts included those salts that form with the carboxylate anion and include salts formed with organic and inorganic cations such as those chosen from the alkali and alkaline earth metals (for example, lithium, sodium, potassium, magnesium, barium and calcium), as well as the ammonium ion and substituted derivatives thereof (for example, dibenzylammonium, benzylammonium, 2-hydroxyethylammonium, and the like). Thus, the term "pharmaceutically acceptable salt" of structures (I) through (VI) is intended to encompass any and all acceptable salt forms.

With regard to stereoisomers, the compounds of structures (I) through (VI) may have chiral centers and may occur as racemates, racemic mixtures and as individual enantiomers or diastereomers. All such isomeric forms are included within the present invention, including mixtures thereof. Furthermore, some of the crystalline forms of the compounds of structures (I) through (VI) may exist as polymorphs, which are included in the present invention. In addition, some of the compounds of structures (I) through (VI) may also form solvates with water or other organic solvents. Such solvates are similarly included within the scope of this invention.

In another embodiment of the invention, pharmaceutical compositions containing one or more compounds of structures (I) through (VI) are disclosed. For the purposes of administration, the compounds of the present invention may be formulated as pharmaceutical compositions. Pharmaceutical compositions of the present invention comprise one or more compounds of the present invention and a pharmaceutically acceptable carrier and/or diluent. The PDE10 inhibitor is present in the composition in an amount which is effective to treat a particular disorder--that is, in an amount sufficient to achieve desired PDE10 inhibition, and preferably with acceptable toxicity to the warm-blooded animal. Typically, the pharmaceutical compositions of the present invention may include a PDE10 inhibitor in an amount from 0.1 mg to 250 mg per dosage depending upon the route of administration, and more typically from 1 mg to 60 mg. Appropriate concentrations and dosages can be readily determined by one skilled in the art.

In general terms, a typical daily dosage might range from about 1 µg/kg to 100 mg/kg, preferably 0.01-100 mg/kg, more preferably 0.1-70 mg/kg, depending on the type and severity of the disease whether, for example, by one or more separate administrations. For repeated administrations over several days or longer, depending on the condition, the treatment is sustained until a desired suppression of disease symptoms occurs. However, other dosage regimens may be useful. The progress of this therapy can be monitored by standard techniques and assays. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Pharmaceutically acceptable carrier and/or diluents are familiar to those skilled in the art. For compositions formulated as liquid solutions, acceptable carriers and/or diluents include saline and sterile water, and may optionally include antioxidants, buffers, bacteriostats and other common additives. The compositions can also be formulated as pills, capsules, granules, or tablets which contain, in addition to a PDE10 inhibitor, diluents, dispersing and surface active agents, binders, and lubricants. One skilled in this art may further formulate the PDE10 inhibitor in an appropriate manner, and in accordance with accepted practices, such as those disclosed in Remington's Pharmaceutical Sciences, Gennaro, Ed., Mack Publishing Co., Easton, PA 1990.

The compound for use in treating schizophrenia includes systemic administration of a PDE10 inhibitor recited in claim 1 of
this invention, preferably in the form of a pharmaceutical composition as discussed above. As used herein, systemic administration includes oral and parenteral methods of administration, including subcutaneous, intramuscular, intracranial, intraorbital, ophthalmic, intraventricular, intracapsular, intraarticular, intraspinal, intracisternal, intraperitoneal, intranasal, aerosol, intravenous, intradermal, inhalational, transdermal, transmucosal, and rectal administration.

For oral administration, suitable pharmaceutical compositions of PDE10 inhibitors include powders, granules, pills, tablets, and capsules as well as liquids, syrups, suspensions, and emulsions. These compositions may also include flavorants, preservatives, suspending, thickening and emulsifying agents, and other pharmaceutically acceptable additives and excipients. For parenteral administration, the compounds of the present invention can be prepared in aqueous injection solutions which may contain, in addition to the PDE10 inhibitor, buffers, antioxidants, bacteriostats, and other additives and excipients commonly employed in such solutions. Compositions of the present invention may be carried in a delivery system to provide for sustained release or enhanced uptake or activity of the therapeutic compound, such as a liposomal or hydrogel system for injection, a microparticle, nanopartical or micelle system for oral or parenteral delivery, or a staged capsule system for oral delivery.

In a further advantage of the present invention, compounds of structures (I) through (VI) are expected to avoid or reduce metabolic side effects associated with conventional antipsychotics, in particular the incidence of therapeutically induced obesity. For example, chronic use of olanzapine (Zyprexa®), the most widely prescribed medication to treat schizophrenia, and related atypical antipsychotics is associated with significant metabolic side effects including obesity and associated conditions such as diabetes.

In animals, subchronic treatment with olanzapine stimulates food intake and increases body weight, consistent with human situations. Furthermore, olanzapine acutely lowers blood leptin levels. Leptin is a satiety hormone produced from adipose tissues, and decrease of leptin level stimulates appetite. It is theorized that olanzapine could stimulate food intake at least partly by reducing leptin levels. Acute administration of olanzapine also changes the animal's response in glucose and insulin levels in glucose tolerance tests, which may also be directly linked to olanzapine's effect in food intake and body weight gain. Examination of the acute effect of PDE10 inhibitors of the present invention on metabolism, such as leptin, insulin and glucose changes during a metabolic challenge in standard animal models, as well as the chronic effect of PDE10 inhibitors of the present invention in food intake, body weight and energy homeostasis, in comparison with olanzapine should provide evidence to the pharmaceutical advantage of PDE10 inhibitors as antipsychotics in terms of less side-effect concerns.

The compositions of the present invention may be administered in combination with one ore more additional therapeutic agents, in combination or by concurrent or sequential administration. Suitable additional agents (*i.e.,* adjuvants) may include typical antipsychotics that block dopamine-D₂ receptors and serotonin 5HT₂ receptors, e.g., haloperidol, fluphenazine, chlorpromazine, and atypical antipsychotics, e.g., clozapine, olanzapine, risperidone, quetiapine, ziprasidone.

Compounds of this invention may be assayed to determine their IC₅₀ values by a modification of the two-step method of Thompson and Appleman (Biochemistry 10; 311-316; 1971). In short, cAMP is spiked with (³H)cAMP and incubated with PDE10 and various concentrations of a compound of structure (I). After the appropriate incubation time, the reaction is terminated by heating. The mixture is then subjected to treatment with snake venom phosphatase. The phosphatase hydrolyzes any AMP in the mixture, but leaves unreacted cAMP intact. Thus, by separating cAMP from the mixture and determining its concentration (by radiography), the percent of inhibition can be determined. IC₅₀ values can be calculated by performing the experiment at several concentrations using standard graphical means. A detailed description of the actual technique used for IC₅₀ assays are shown in Example 8. To this end, PDE10 inhibitors of the invention have an IC₅₀ of 100µM or less, generally less than 10 µM, and typically less than 1 µM.

### EXAMPLES

### EXAMPLE 1

### (E)-2-(3-OXO-3,4-DIHYDRO-2H-BENZO[B][1,4]THIAZIN-2-YL)-N'-(3,4,5-TRIMETHOXYBENZYLIDENE)ACETOHYDRAZIDE

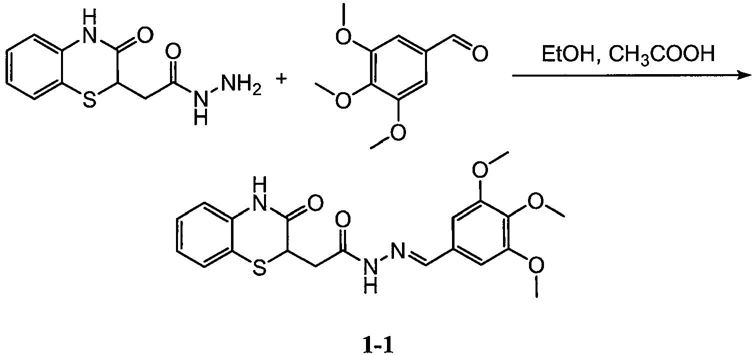

In a round-bottom glass flask equipped with a magnetic stir bar (3-Oxo-3, 4-dihydro-2*H*-benzo [1, 4] thiazin-2-yl)-acetic acid hydrazide (1 eq. 0.063 mmol; 15 mg) was dissolved in ethanol (3 mL) at room temperature. To this well stirred solution, acetic acid (∼3 drops) and 3, 4, 5-trimethoxy-benzaldehyde solution (0.063 mmol; 12.4 mg dissolved in 0.5 mL of ethanol) were added, and the reaction mixture was stirred for another 5 hours at ambient temperature and monitored by TLC (typically with 1,2-dichloroethane-ethanol 5:1). The mixture was then concentrated under vacuum using a rotary evaporator. The crude product was diluted with hexanes and triturated. The precipitated solid product was transferred to a glass fiber funnel, the supernatant was removed by vacuum filtration and the solid was washed thoroughly with *n*-hexane twice to yield: 16 mg (61%) of 1-1.

### EXAMPLE 2

### (E)-2-(1H-INDOL-3-YL)-N'-(3,4,5-TRIMETHOXYBENZYLIDENE)ACETOHYDRAZIDE

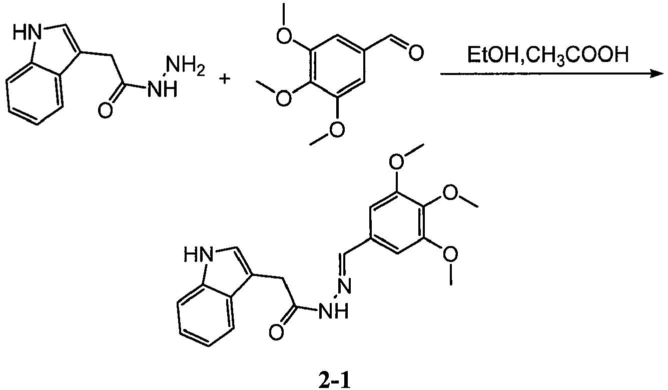

In a round-bottom glass flask equipped with a magnetic stir bar (1*H-*indol-3-yl)-acetic acid hydrazide (1 eq. 0.793 mmol; 150 mg) was dissolved in ethanol (10 mL) at room temperature. To this well stirred solution, acetic acid (∼3 mL) and the 3, 4, 5-trimethoxy-benzaldehyde solution (1 eq. 0.793 mmol; 155.5 mg dissolved in 5mL of ethanol) were added dropwise. The reaction mixture was stirred at room temperature until the reaction was complete (approximately 2 hours) as determined by TLC (typically with 1,2-dichloroethane-ethanol 5:1). The mixture was then concentrated under reduced pressure. The crude product was diluted with hexanes and triturated. The precipitated solid product was transferred to a glass fiber funnel, the supernatant was removed by vacuum filtration and the solid was washed thoroughly with *n*-hexane twice to yield 104 mg (36%) of **2-1.**

Referring to Table 1 below, the listed derivatives of structure (II), where R³, R⁴ and R⁵ are hydrogen, were synthesized according to the procedures outlined in Examples 1 and 2.

**Table 1**

| | | | | | |
|---|---|---|---|---|---|
| 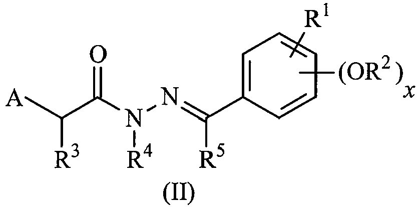 | | | | | |

| No. | MW | A | R¹ | *x* | R² |
|---|---|---|---|---|---|
| 1-1 | 415.47 | 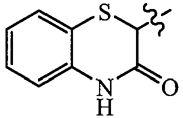 | H | 3 | 3-CH₃ |
| | | | | | 4-CH₃ |
| | | | | | 5-CH₃ |
| 2-1 | 367.40 | 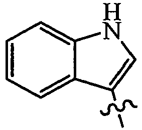 | H | 3 | 3-CH₃ |
| | | | | | 4-CH₃ |
| | | | | | 5-CH₃ |
| 2-2 | 403.38 | 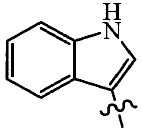 | H | 3 | 3-CH₃ |
| | | | | | 4-CHF₂ |
| | | | | | 5-CH₃ |
| 2-3 | 367.40 | 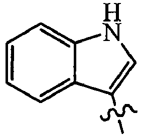 | H | 3 | 2-CH₃ |
| | | | | | 4-CH₃ |
| | | | | | 5-CH₃ |
| 2-4 | 415.47 | 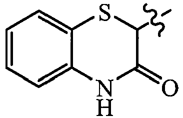 | H | 3 | 2-CH₃ |
| | | | | | 4-CH₃ |
| | | | | | 5-CH₃ |
| 2-5 | 355.37 | 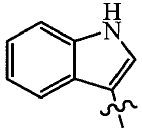 | 2-F | 2 | 4-CH₃ |
| | | | | | 5-CH₃ |
| 2-6 | 410.43 | 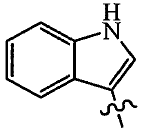 | H | 3 | 3-CH₃ |
| | | | | | 4-CH₂CONH₂ |
| | | | | | 5-CH₃ |
| 2-7 | 451.45 | 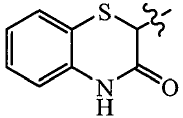 | H | 3 | 3-CH₃ |
| | | | | | 4-CHF₂ |
| | | | | | 5-CH₃ |
| 2-8 | 458.49 | 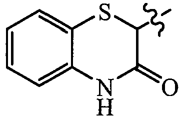 | H | 3 | 3-CH₃ |
| | | | | | 4-CH₂CONH₂ |
| | | | | | 5-CH₃ |
| 2-9 | 351.41 | 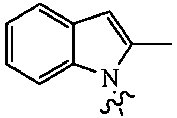 | H | 2 | 3-CH₃ |
| | | | | | 4-CH₃ |
| 2-10 | 338.37 | 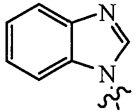 | H | 2 | 3-CH₃ |
| | | | | | 4-CH₃ |
| 2-11 | 332.38 | 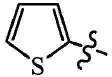 | H | 2 | 3-CH₃ |
| | | | | | 2-COCH₃ |
| 2-12 | 339.35 | 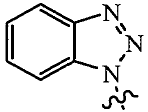 | H | 2 | 3-CH₃ |
| | | | | | 4-CH₃ |
| 2-13 | 333.43 | 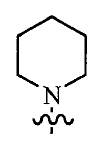 | H | 2 | 3-CH₂CH₃ |
| | | | | | 4-CH₂CH₃ |
| 2-14 | 488.54 | 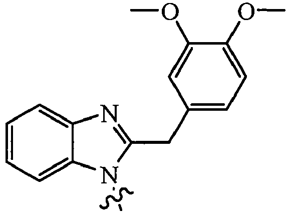 | H | 2 | 3-CH₃ |
| | | | | | 4-CH₃ |
| 2-15 | 369.40 | 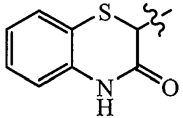 | H | 2 | 3-CH₂-4 |
| 2-16 | 371.41 | 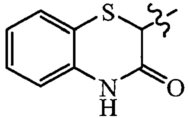 | H | 2 | 3-H |
| | | | | | 4-CH₃ |
| 2-17 | 385.44 | 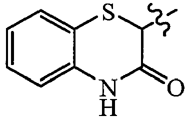 | H | 2 | 3-CH₂CH₃ |
| | | | | | 4-H |
| 2-18 | 489.57 | 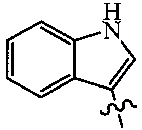 | H | 2 | 3-benzyl |
| | | | | | 4-benzyl |
| 2-19 | 321.34 | 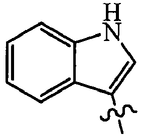 | H | 2 | 3-CH₂-4 |
| 2-20 | 367.40 | 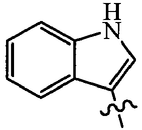 | H | 3 | 2-CH₃ |
| | | | | | 3-CH₃ |
| | | | | | 4-CH₃ |
| 2-21 | 491.57 | 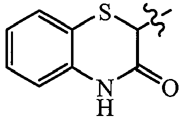 | H | 3 | 3-CH₃ |
| | | | | | 4-benzyl |
| | | | | | 5-CH₃ |
| 2-22 | 443.50 | 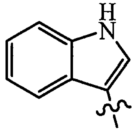 | H | 3 | 3-CH₃ |
| | | | | | 4-benzyl |
| | | | | | 5-CH₃ |
| 2-23 | 415.47 | 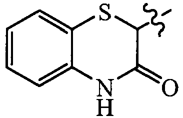 | H | 3 | 2-CH₃ |
| | | | | | 3-CH₃ |
| | | | | | 4-CH₃ |
| 2-24 | 537.64 | 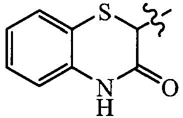 | H | 2 | 3-benzyl |
| | | | | | 4-benzyl |

As used in Table 1 above, as well as in the tables that follow, the numbers preceding (and following in the case of alkanediyls) the R¹ and R² substituents refer to the substituent's position on the aromatic ring as shown below.

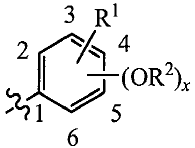

### EXAMPLE 3

### (E)-N-(2-(2-(4-HYDROXY-3-METHOXYBENZYLIDENE)HYDRAZINYL)-2-OXOETHYL)ISONICOTINAMIDE

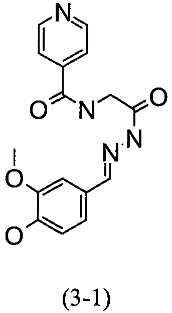

Referring to Table 2, the above derivative of structure (III), where R¹, R³, R⁴ and R⁵ are hydrogen, can be synthesized by Reaction Scheme 2 from corresponding intermediate 5.

**Table 2**

| | | | | | | | |
|---|---|---|---|---|---|---|---|
| 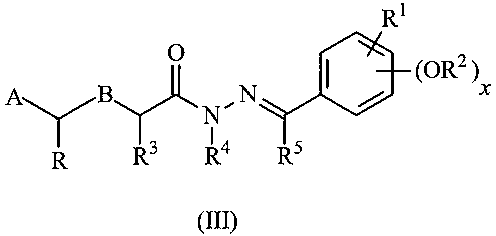 | | | | | | | |

| No. | MW | A | B | R | R⁶ | x | R² |
|---|---|---|---|---|---|---|---|
| 3-1 | 328.32 | 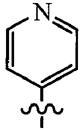 | NR⁶ | =O | H | 2 | 3-CH₃ |
| | | | | | | | 4-H |

### EXAMPLE 4

### (E)-2-(1-BENZYL-1H-BENZO[D]IMIDAZOL-2-YLTHIO)-N'-(4-ETHOXY-3-METHOXYBENZYLIDENE)ACETOHYDRAZIDE

### Step 4A: Preparation of ethyl 2-(1H-benzo[d]imidazol-2-ylthio)acetate 4a

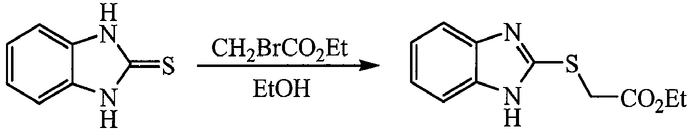

To a stirred solution of 1*H*-benzo[*d*]imidazole-2(3*H*)-thione (20.0 g, 0.13 mol, 1.0 eq.) in anhydrous ethanol (500 mL) at room temperature, was added potassium carbonate (9.25 g, 0.07 mol, 0.5 eq.) and ethyl bromoacetate (26.7 g, 0.16 mmol, 1.2 eq.). The reaction was heated to reflux and stirred at that temperature for 6 hours. The solvent was removed *in vacuo* and water (200 mL) was added. The aqueous phase was extracted with dichloromethane (3 x 100 mL), and the combined organic extracts were washed with water (200 mL) and brine (200 mL). The organic phase was dried over magnesium sulfate and *concentrated in vacuo* to give a colorless oil which solidified on standing. Purification by flash chromatography (CHCl₃ → 10% MeOH:90% CHCl₃) gave **4a** as a white solid (11.7 g, 37%).

### Step 4B: Preparation of ethyl 2-(1-benzyl-1H-benzo[d]imidazol-2-ylthio)acetate 4b

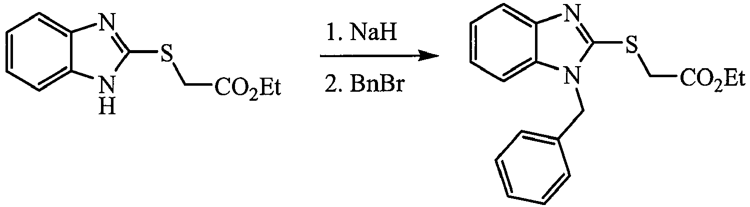

To a stirred solution of ethyl 2-(1*H*-benzo[*d*]imidazol-2-ylthio)acetate **4a** (11.2 g, 47.6 mmol, 1.0 eq.) in 10:1 diethyl ether:dimethylformamide (450 mL) at 0 °C, was added 60% sodium hydride in mineral oil (2.09 g, 52.3 mmol, 1.1 eq.), and the reaction was stirred at 0 °C for 20 minutes. Benzyl bromide (9.78 g, 57.1 mmol, 1.2 eq.) was added at 0 °C, and the reaction was stirred with warming to room temperature for 16 hours. The reaction mixture was quenched with methanol (100 mL) and *concentrated in vacuo.* The residue was taken into hot chloroform (400 mL) and filtered. The filtrate was washed with water (200 mL), 1M HCl (200 mL) and brine (200 mL). The organic layer was dried over magnesium sulfate and concentrated *in vacuo.* Purification by flash chromatography (10% EtOAc:90% petroleum ether) gave **4b** as a pale-yellow solid (4.82 g, 30%).

### Step 4C: Preparation of 2-(1-benzyl-1H-benzo[d]imidazol-2-ylthio)acetohydrazide 4c

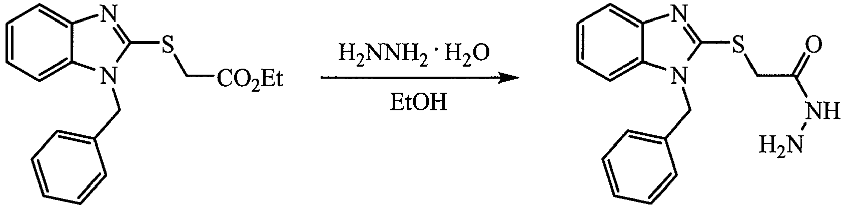

To a stirred solution of ethyl 2-(1-benzyl-1*H-*benzo[*d*]imidazol-2-ylthio)acetate **4b** (200 mg, 0.61 mmol, 1.0 eq.) in ethanol (5 mL), was added hydrazine monohydrate (61 mg, 1.23 mmol, 2.0 eq.), and the reaction mixture was heated to reflux and stirred at that temperature for 16 hours. The reaction mixture was cooled to room temperature and *concentrated in vacuo.* The mixture was dissolved in ethyl acetate (20 mL) and washed with water (20 mL) and brine (20 mL). The organic layer was dried over magnesium sulfate and concentrated *in vacuo* giving **4c** as a pale yellow solid which was used without further purification (200 mg, 100%).

### Step 4D Preparation of (E)-2-(1-benzyl-1H-benzo[d]limidazol-2-ylthio)-N'-(4-ethoxy-3-methoxybenzylidene)acetohydrazide 4-1

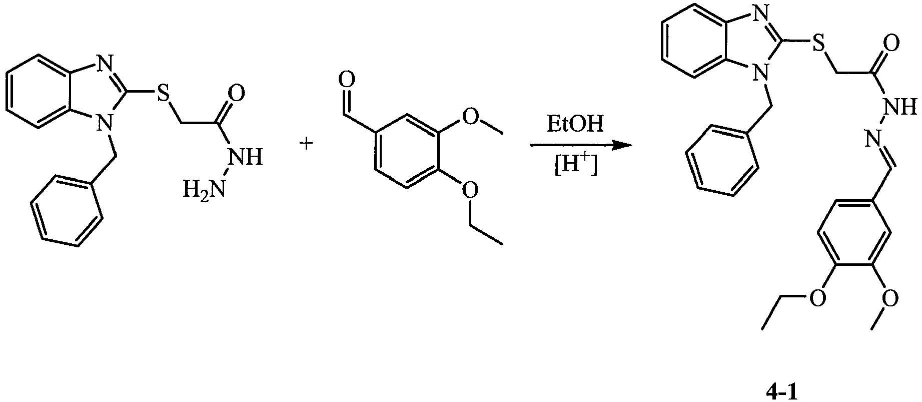

To a stirred solution of 2-(1-benzyl-1*H*-benzo[*d*]imidazol-2-ylthio)acetohydrazide **4c** (200 mg, 0.61 mmol, 1.0 eq.) in ethanol (5 mL), was added 3-methoxy-4-ethoxybenzaldehyde (110 mg, 0.61 mmol, 1.0 eq.) and acetic acid (3 drops). The mixture was heated to reflux and stirred at that temperature for 16 hours. The reaction mixture was cooled to room temperature, and the precipitate that formed on cooling was filtered and washed with water (10 mL), ethanol (10 mL) and diethyl ether (10 mL). The solid was dried by high vacuum overnight giving **4-1** as a white solid (196 mg, 67%).

### EXAMPLE 5

### (E)-N'-(3,4-DIMETHOXYBENZYLIDENE)-2-(2-METHYLQUINOLIN-4-YLTHIO)ACETOHYDRAZIDE HYDROCHLORIDE

### Step 5A Preparation of 2-methylquinoline-4-thiol 5a

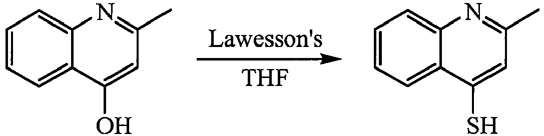

A stirred suspension of 2-methylquinolin-4-ol (5.10 g, 32.0 mmol, 1.0 eq.) in tetrahydrofuran (51 mL) was heated to 50 °C, and 6.5g of Lawesson's Reagent (6.5 g, 16.0 mmol, 0.5 eq.) was added in one portion. The reaction was heated to 80 °C and stirred at that temperature for 6 hours. The reaction was poured into a hot biphasic solution of ethyl acetate (200 mL) and water (200 mL) and was vigorously stirred. A viscous orange gel precipitated from the solution. The water/ethyl acetate solution was decanted into a separating funnel and the organic layer isolated. The aqueous layer was extracted with ethyl acetate (4 x 200ml), and the combined extracts were dried over magnesium sulfate. The solution was filtered and concentrated *in vacuo.* The resulting orange gel was purified by flash chromatography (50% EtOAc:50% petroleum ether → 100% EtOAc) to yield **5a** as a yellow solid (1.70 g, 30%).

### Step 5B Preparation of methyl 2-(2-methylquinolin-4-ylthio)acetate 5b

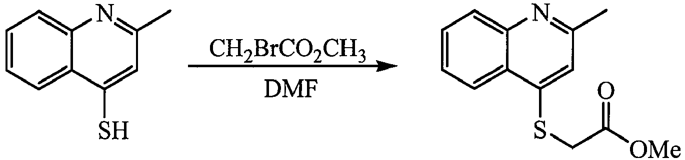

2-methylquinoline-4-thiol **5a** (1.00 g, 5.71mmol, 1.00 eq.) was dissolved in dimethylformamide (10 mL) at room temperature. Potassium carbonate (0.87 g, 6.28 mmol, 1.10 eq.) was added and the mixture was stirred for 15 minutes. Methyl bromoacetate (921 mg, 6.02 mmol, 1.05 eq.) was added dropwise to the solution, and the reaction was stirred at room temperature for 2.5 hours. Water (20 mL) was added to the reaction mixture, and the aqueous layer was extracted with ethyl acetate (30 mL). The aqueous layer was further extracted with ethyl acetate (3 x 50 mL), and the combined organic layers were dried over magnesium sulfate, filtered and concentrated *in vacuo.* The resulting residue was dried on high vacuum to yield 5b as a brown solid (1.25 g, 88%).

### Step 5C Preparation of 2-(2-methylquinolin-4-ylthio)acetohydrazide 5c

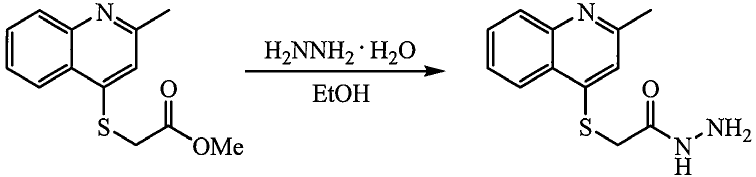

Example 5, Step 5C was performed in an analogous fashion to Example 4, Step 4C.

### Step 5D Preparation of (E)-N'-(3,4-dimethoxybenzylidene)-2-(2-methylquinolin-4-ylthio)acetohydrazide 5-1

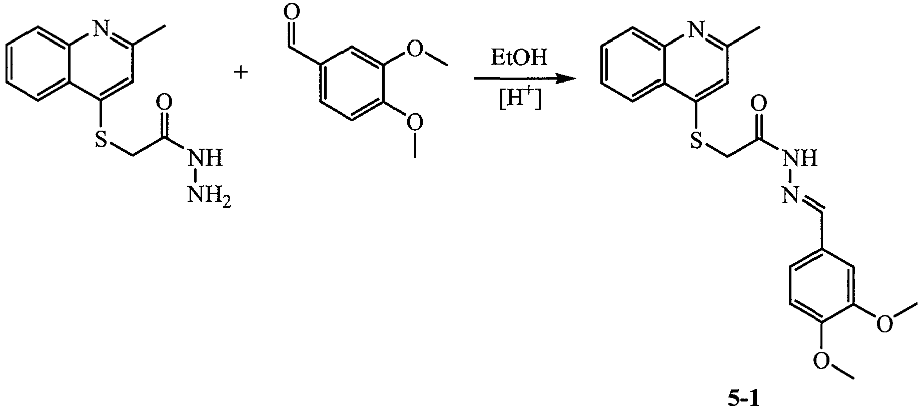

Example 5, Step 5D was performed in an analogous fashion to Example 4, Step 4D.

### Step 5E Preparation of (E)-N'-(3,4-dimethoxybenzylidene)-2-(2-methylquinolin-4-ylthio)acetohydrazide hydrochloride 5-1a

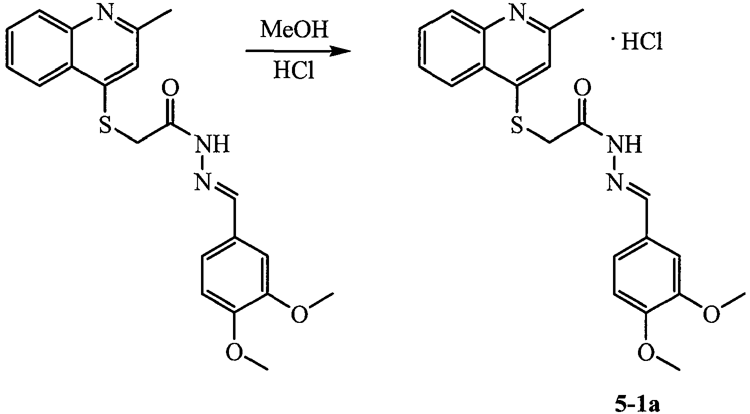

2-(2-methylquinolin-4-ylsulfanyl)-acetic acid-(3,4-dimethoxybenzylidene)-hydrazide 5-1 (220 mg, 0.56 mmol, 1.0 eq.) in methanol (40 mL) and dimethylsulfoxide (2 mL) was heated to 100 °C for 2 hours until completely dissolved before being allowed to cool to 50 °C. 1.25 M methanolic hydrochloric acid (1 eq.) was then added, and the mixture was stirred for 20 min before partially *concentrating in vacuo.* The product was then obtained through filtration and dried to give **5-1a** (180 mg, 75%).

Referring to Table 3, the following derivatives of structure (IV), wherein R³ is hydrogen, were synthesized according to the procedures outlined in Examples 4 and 5 (in the following table, an R¹ substituent is absent when H is designated).

**Table 3**

| | | | | | | | | | |
|---|---|---|---|---|---|---|---|---|---|
| 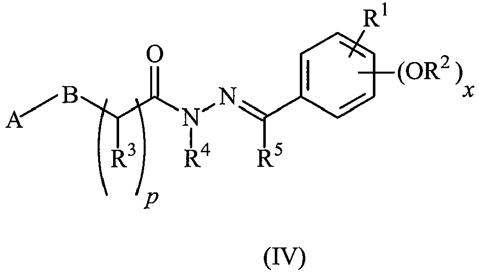 | | | | | | | | | |

| No. | MW | A | B | R¹ | p | *x* | R² | R⁴ | R⁵ |
|---|---|---|---|---|---|---|---|---|---|
| 4-1 | 474.58 | 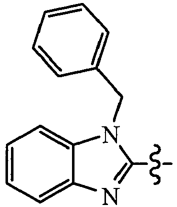 | S | H | 1 | 2 | 3-CH₃ | H | H |
| | | | | | | | 4-CH₂CH₃ | | |
| 5-1 | 395.48 | 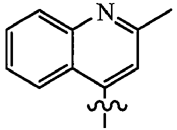 | S | H | 1 | 2 | 3-CH₃ | H | H |
| | | | | | | | 4-CH₃ | | |
| 5-2 | 526.56 | 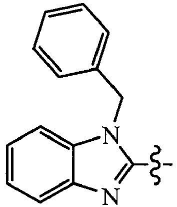 | S | H | 1 | 3 | 3-CH₃ | H | H |
| | | | | | | | 4-CHF₂ | | |
| | | | | | | | 5-CH₃ | | |
| 5-3 | 561.01 | 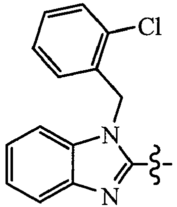 | S | H | 1 | 3 | 3-CH₃ | H | H |
| | | | | | | | 4-CHF₂ | | |
| | | | | | | | 5-CH₃ | | |
| 5-4 | 512.99 | 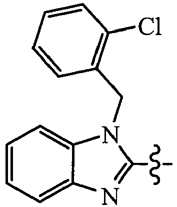 | S | 2-F | 1 | 2 | 4-CH₃ | H | H |
| | | | | | | | 5-CH₃ | | |
| 5-5 | 525.03 | 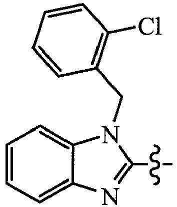 | S | H | 1 | 3 | 2-CH₃ | H | H |
| | | | | | | | 4-CH₃ | | |
| | | | | | | | 5-CH₃ | | |
| 5-6 | 525.03 | 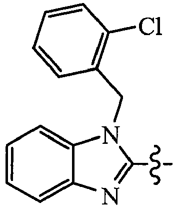 | S | H | 1 | 3 | 3-CH₂CH₃ | H | H |
| | | | | | | | 4-H | | |
| 5-7 | 478.55 | 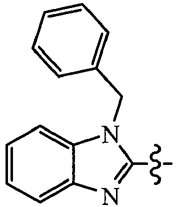 | S | 2-F | 1 | 2 | 4-CH₃ | H | H |
| | | | | | | | 5-CH₃ | | |
| 5-8 | 460.56 | 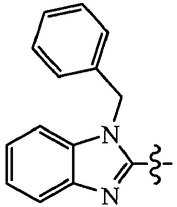 | S | H | 1 | 2 | 3-CH₂CH₃ | H | H |
| | | | | | | | 4-H | | |
| 5-9 | 490.58 | 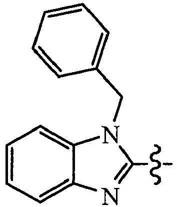 | S | H | 1 | 3 | 2-CH₃ | H | H |
| | | | | | | | 4-CH₃ | | |
| | | | | | | | 5-CH₃ | | |
| 5-10 | 533.61 | 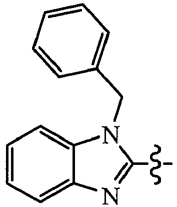 | S | H | 1 | 3 | 3-CH₃ | H | H |
| | | | | | | | 4-CH₂CONH₂ | | |
| | | | | | | | 5-CH₃ | | |
| 5-11 | 460.56 | 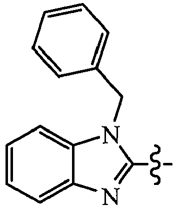 | S | H | 1 | 2 | 2-CH₃ | H | H |
| | | | | | | | 4-CH₃ | | |
| 5-12 | 411.48 | 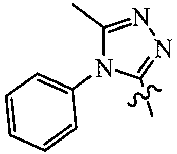 | S | H | 1 | 2 | 3-CH₃ | H | H |
| | | | | | | | 4-CH₃ | | |
| 5-13 | 360.44 | 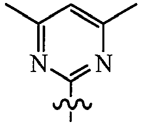 | S | H | 1 | 2 | 3-CH₃ | H | H |
| | | | | | | | 4-CH₃ | | |
| 5-14 | 473.55 | 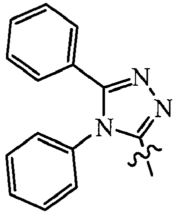 | S | H | 1 | 2 | 3-CH₃ | H | H |
| | | | | | | | 4-CH₃ | | |
| 5-15 | 331.39 | 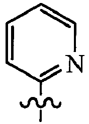 | S | H | 1 | 2 | 3-CH₃ | H | H |
| | | | | | | | 4-CH₃ | | |
| 5-16 | 384.46 | 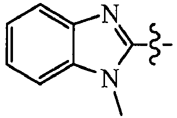 | S | H | 1 | 2 | 3-CH₃ | H | H |
| | | | | | | | 4-CH₃ | | |
| 5-17 | 381.45 | 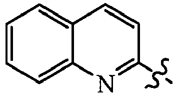 | S | H | 1 | 2 | 3-CH₃ | H | H |
| | | | | | | | 4-CH₃ | | |
| 5-18 | 387.48 | 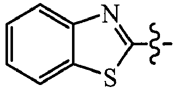 | S | H | 1 | 2 | 2-CH₃ | H | H |
| | | | | | | | 3-CH₃ | | |
| 5-19 | 430.53 | 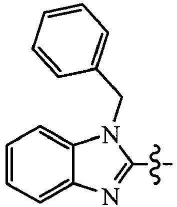 | S | H | 1 | 1 | 4-CH₃ | H | H |
| 5-20 | 331.39 | 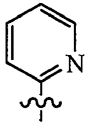 | S | H | 1 | 2 | 3-H | H | H |
| | | | | | | | 4-CH₂CH₃ | | |
| 5-21 | 361.42 | 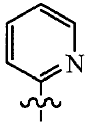 | S | H | 1 | 3 | 3-CH₃ | H | H |
| | | | | | | | 4-CH₃ | | |
| | | | | | | | 5-CH₃ | | |
| 5-22 | 459.53 | 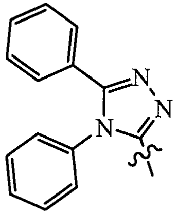 | S | H | 1 | 2 | 3-CH₃ | H | H |
| | | | | | | | 4-H | | |
| 5-23 | 432.50 | 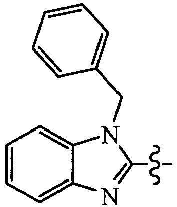 | S | H | 1 | 2 | 3-H | H | H |
| | | | | | | | 4-H | | |
| 5-24 | 370.43 | 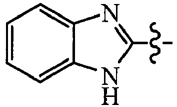 | S | H | 1 | 2 | 3-CH₃ | H | H |
| | | | | | | | 4-CH₃ | | |
| 5-25 | 490.58 | 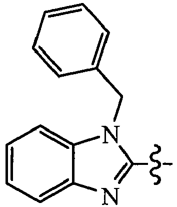 | S | H | 1 | 3 | 3-CH₃ | H | H |
| | | | | | | | 4-CH₃ | | |
| | | | | | | | 5-CH₃ | | |
| 5-26 | 460.56 | 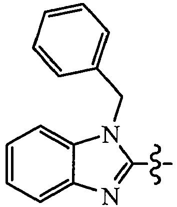 | S | H | 1 | 2 | 3-CH₃ | H | H |
| | | | | | | | 4-CH₃ | | |
| 5-27 | 495.00 | 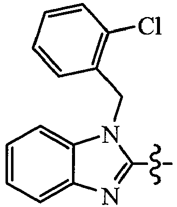 | S | H | 1 | 2 | 3-CH₃ | H | H |
| | | | | | | | 4-CH₃ | | |
| 5-28 | 474.54 | 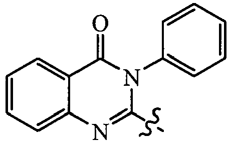 | S | H | 1 | 2 | 3-CH₃ | H | H |
| | | | | | | | 4-CH₃ | | |
| 5-29 | 409.51 | 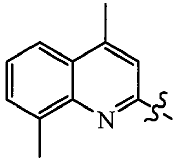 | S | H | 1 | 2 | 3-CH₃ | H | H |
| | | | | | | | 4-CH₃ | | |
| 5-30 | 387.48 | 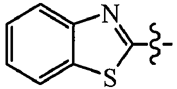 | S | H | 1 | 2 | 3-CH₃ | H | H |
| | | | | | | | 4-CH₃ | | |
| 5-31 | 474.58 | 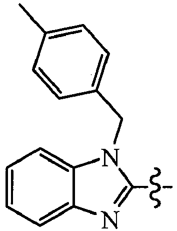 | S | H | 1 | 2 | 3-CH₃ | H | H |
| | | | | | | | 4-CH₃ | | |
| 5-32 | 371.41 | 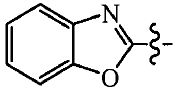 | S | H | 1 | 2 | 3-CH₃ | H | H |
| | | | | | | | 4-CH₃ | | |
| 5-33 | 495.00 | 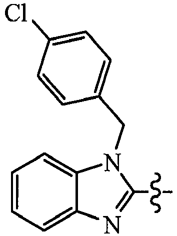 | S | H | 1 | 2 | 3-CH₃ | H | H |
| | | | | | | | 4-CH₃ | | |
| 5-34 | 423.47 | 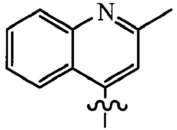 | O | H | 1 | 3 | 3-CH₃ | H | H |
| | | | | | | | 4-CH₂CH₃ | | |
| | | | | | | | 5-CH₃ | | |
| 5-35 | 446.53 | 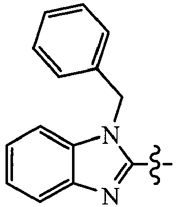 | S | H | 1 | 2 | 3-CH₃ | H | H |
| | | | | | | | 4-H | | |
| 5-35 | 398.48 | 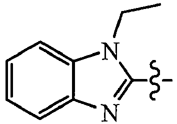 | S | H | 1 | 2 | 3-CH₃ | H | H |
| | | | | | | | 4-CH₃ | | |
| 5-37 | 379.42 | 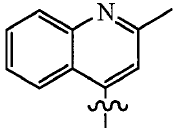 | O | H | 1 | 2 | 3-CH₃ | H | H |
| | | | | | | | 4-CH₃ | | |
| 5-38 | 433.51 | 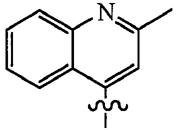 | O | H | 1 | 2 | 3-CH₃ | H | H |
| | | | | | | | 4-cyclopentyl | | |
| 5-39 | 504.61 | 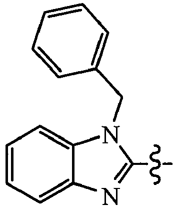 | S | H | 1 | 3 | 3-CH₃ | H | H |
| | | | | | | | 4-CH₂CH₃ | | |
| | | | | | | | 5-CH₃ | | |
| 5-40 | 488.61 | 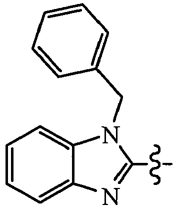 | S | H | 1 | 2 | 3-CH₃ | H | |
| | | | | | | | 4-*n*-propyl | | H |
| 5-41 | 488.61 | 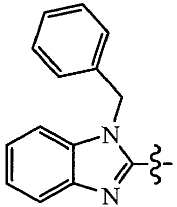 | S | H | 1 | 2 | 3-CH₃CH₃ | H | H |
| | | | | | | | 4-CH₂CH₃ | | |
| 5-42 | 463.53 | 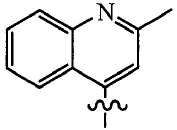 | O | H | 1 | 3 | 3-CH₃ | H | H |
| | | | | | | | 4-cyclopentyl | | |
| | | | | | | | 5-CH₃ | | |
| 5-43 | 486.59 | 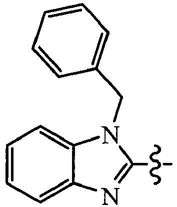 | S | H | 1 | 2 | 3-CH₃ | H | H |
| | | | | | | | 4-(2)-propenyl | | |
| 5-44 | 474.58 | 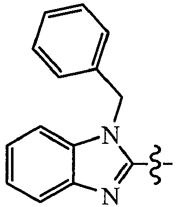 | S | H | 1 | 2 | 3-CH₃ | H | H |
| | | | | | | | 4-CH₂CH₃ | | |
| 5-45 | 423.53 | 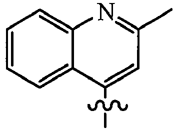 | S | H | 1 | 2 | 3-CH₃ | H | H |
| | | | | | | | 4-*n*-propyl | | |
| 5-46 | 407.47 | 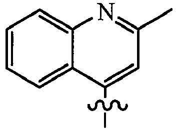 | O | H | 1 | 2 | 3-CH₃ | H | H |
| | | | | | | | 4-*n*-propyl | | |
| 5-47 | 514.65 | 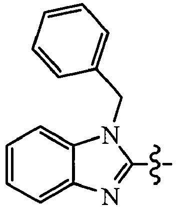 | S | H | 1 | 2 | 3-cyclopentyl | H | H |
| | | | | | | | 4-CH₃ | | |
| 5-48 | 444.51 | 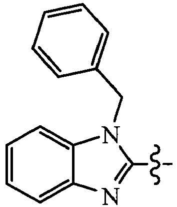 | S | H | 1 | 2 | 3-CH₂-4 | H | H |
| 5-49 | 514.65 | 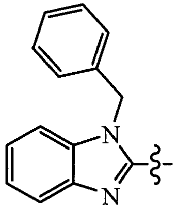 | S | H | 1 | 2 | 3-CH₃ | H | H |
| | | | | | | | 4-cyclopentyl | | |
| 5-50 | 518.64 | 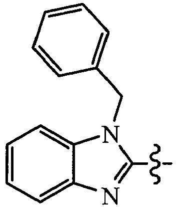 | S | H | 1 | 3 | 3-CH₃ | H | H |
| | | | | | | | 4-*n*-propyl | | |
| | | | | | | | 5-CH₃ | | |
| 5-51 | 544.67 | 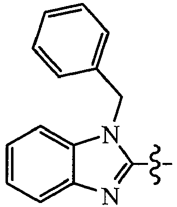 | S | H | 1 | 3 | 3-CH₃ | H | H |
| | | | | | | | 4-cyclopentyl | | |
| | | | | | | | 5-CH₃ | | |
| 5-52 | 612.75 | 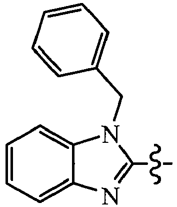 | S | H | 1 | 2 | 3-benzyl | H | H |
| | | | | | | | 4-benzyl | | |
| 5-53 | 446.53 | 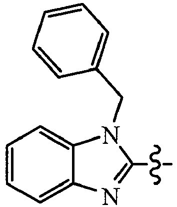 | S | H | 1 | 2 | 3-H | H | H |
| | | | | | | | 4-CH₃ | | |
| 5-54 | 490.58 | 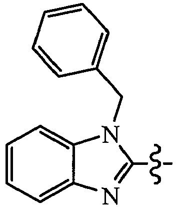 | S | H | 1 | 3 | 2-CH₃ | H | H |
| | | | | | | | 3-CH₃ | | |
| | | | | | | | 4-CH₃ | | |
| 5-55 | 566.68 | 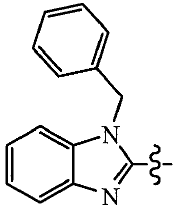 | S | H | 1 | 3 | 3-CH₃ | H | H |
| | | | | | | | 4-benzyl | | |
| | | | | | | | 5-CH₃ | | |
| 5-56 | 647.20 | 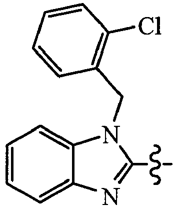 | S | H | 1 | 2 | 3-benzyl | H | H |
| | | | | | | | 4-benzyl | | |
| 5-57 | 478.96 | 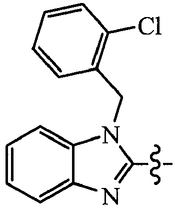 | S | H | 1 | 2 | 3-CH₂-4 | H | H |
| 5-58 | 480.97 | 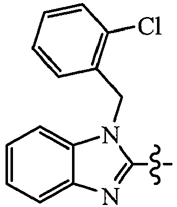 | S | H | 1 | 2 | 3-H | H | H |
| | | | | | | | 4-CH₃ | | |
| 5-59 | 525.03 | 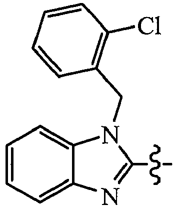 | S | H | 1 | 3 | 2-CH₃ | H | H |
| | | | | | | | 3-CH₃ | | |
| | | | | | | | 4-CH₃ | | |
| 5-60 | 601.12 | 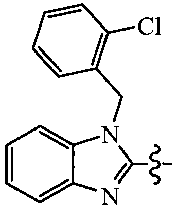 | S | H | 1 | 3 | 3-CH₃ | H | H |
| | | | | | | | 4-benzyl | | |
| | | | | | | | 5-CH₃ | | |
| 5-61 | 456.55 | 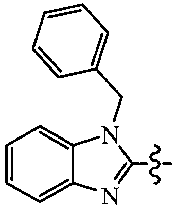 | CH2 | H | 1 | 2 | 3-*n*-propyl | H | H |
| | | | | | | | 4-CH₃ | | |
| 5-62 | 486.59 | 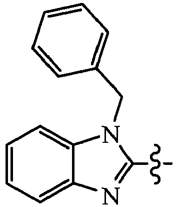 | CH2 | H | 1 | 2 | 3-CH2CH3 | H | H |
| | | | | | | | 4-CH₃ | | |
| 5-63 | 460.56 | 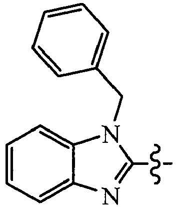 | S | H | 1 | 2 | 3-CH₃ | H | H |
| | | | | | | | 5-CH₃ | | |
| 5-64 | 476.55 | 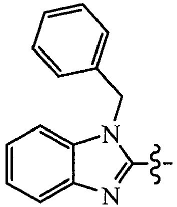 | S | H | 1 | 3 | 3-CH₃ | H | H |
| | | | | | | | 4-H | | |
| | | | | | | | 5-CH₃ | | |
| 5-65 | 486.59 | 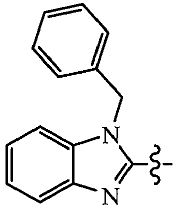 | S | H | 1 | 2 | 3-cyclopropyl | H | H |
| | | | | | | | 4-CH₃ | | |
| 5-66 | 464.97 | 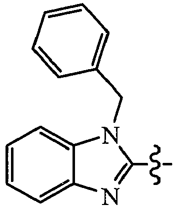 | S | 3-Cl | 1 | 1 | 4-CH₃ | H | H |
| 5-67 | 474.58 | 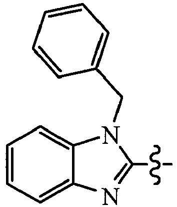 | S | H | 1 | 2 | 3-CH₂CH₃ | H | H |
| | | | | | | | 4-CH₃ | | |
| 5-68 | 446.53 | 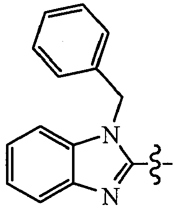 | S | H | 1 | 2 | 3-H | H | H |
| | | | | | | | 4-CH₃ | | |
| 5-69 | 409.51 | 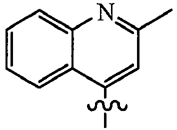 | S | H | 1 | 2 | 3-CH₃ | H | CH₃ |
| | | | | | | | 4-CH₃ | | |
| 5-70 | 365.45 | 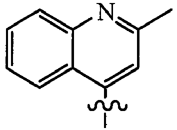 | S | H | 1 | 1 | 4-CH₃ | H | H |
| 5-71 | 474.38 | 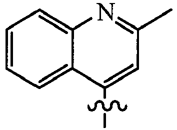 | S | 3-Br | 1 | 2 | 4-H | H | H |
| | | | | | | | 5-CH₂CH₃ | | |
| 5-72 | 349.39 | 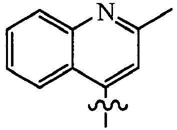 | O | H | 1 | 1 | 2-CH₃ | H | H |
| 5-73 | 395.48 | 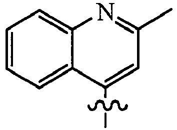 | S | 5-CH₃ | 1 | 2 | 3-CH₃ | H | H |
| | | | | | | | 4-H | | |
| 5-74 | 423.47 | 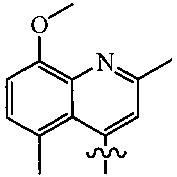 | O | H | 1 | 2 | 3-CH₃ | H | H |
| | | | | | | | 4-CH₃ | | |
| 5-75 | 409.46 | 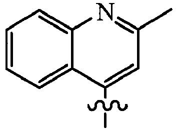 | S | H | 1 | 3 | 3,4-CH₂-5-CH₃ | H | H |
| 5-76 | 429.93 | 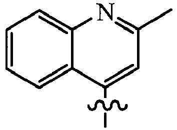 | S | 3-Cl | 1 | 2 | 4-CH₃ | H | H |
| | | | | | | | 5-CH₃ | | |
| 5-77 | 474.38 | 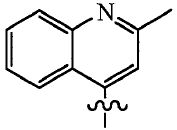 | S | 3-Br | 1 | 2 | 4-CH₃ | H | H |
| | | | | | | | 5-CH₃ | | |
| 5-78 | 409.44 | 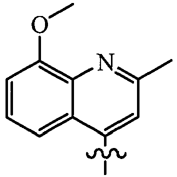 | O | H | 1 | 2 | 3-CH₃ | H | H |
| | | | | | | | 4-CH₃ | | |
| 5-79 | 415.46 | 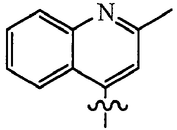 | S | 4-CHF₂ | 1 | 1 | 3-CH₃ | H | H |
| 5-80 | 365.39 | 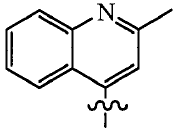 | O | H | 1 | 2 | 3-CH₃ | H | H |
| | | | | | | | 4-H | | |
| 5-81 | 363.42 | 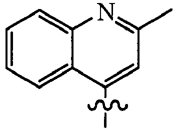 | O | 3-CH₃ | 1 | 1 | 4-CH₃ | H | H |
| 5-82 | 439.47 | 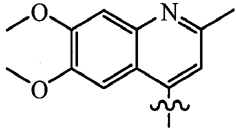 | O | H | 1 | 2 | 3-CH₃ | H | H |
| | | | | | | | 4-CH₃ | | |
| 5-83 | 378.43 | 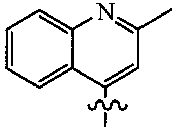 | NR⁶ (R⁶= H) | H | 1 | 2 | 3-CH₃ | H | H |
| | | | | | | | 4-CH₃ | | |
| 5-84 | 365.39 | 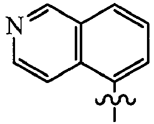 | O | H | 1 | 2 | 3-CH₃ | H | H |
| | | | | | | | 4-CH₃ | | |
| 5-85 | 444.29 | 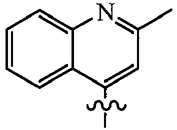 | O | 2-Br | 1 | 2 | 3-H | H | H |
| | | | | | | | 4-CH₃ | | |
| 5-86 | 393.40 | 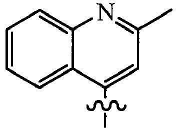 | O | H | 1 | 3 | 3-CH₂-4 | H | H |
| | | | | | | | 5-CH₃ | | |
| 5-87 | 397.41 | 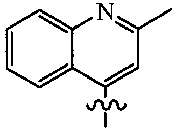 | O | 2-F | 1 | 2 | 4-CH₃ | H | H |
| | | | | | | | 5-CH₃ | | |
| 5-88 | 367.38 | 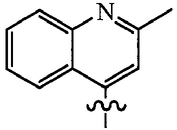 | O | 4-F | 1 | 1 | 3-CH₃ | H | H |
| 5-89 | 399.83 | 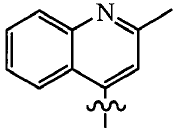 | O | 3-Cl | 1 | 2 | 4-H | H | H |
| | | | | | | | 5-CH₃ | | |
| 5-90 | 458.32 | 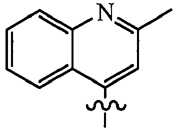 | O | 3-Br | 1 | 2 | 4-H | H | H |
| | | | | | | | 5-CH₂CH₃ | | |
| 5-91 | 413.86 | 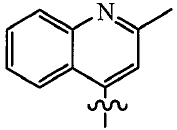 | O | 3-Cl | 1 | 2 | 4-CH₃ | H | H |
| | | | | | | | 5-CH₃ | | |
| 5-92 | 458.32 | 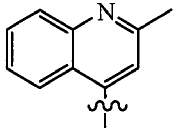 | O | 3-Br | 1 | 2 | 4-CH₃ | H | H |
| | | | | | | | 5-CH₃ | | |
| 5-93 | 371.41 | 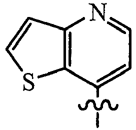 | O | H | 1 | 2 | 3-CH₃ | H | H |
| | | | | | | | 4-CH₃ | | |
| 5-94 | 383.38 | 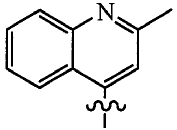 | O | 3-F | 1 | 2 | 4-H | H | H |
| | | | | | | | 5-CH₃ | | |
| 5-95 | 383.83 | 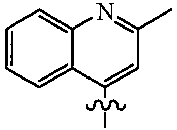 | O | 3-Cl | 1 | 1 | 4-CH₃ | H | H |
| 5-96 | 429.42 | 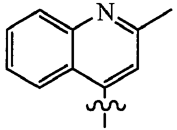 | O | H | 1 | 2 | 3-CH₂CH₃ | H | H |
| | | | | | | | 4-CHF₂ | | |
| 5-97 | 415.40 | 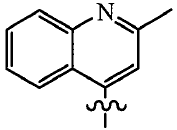 | O | H | 1 | 2 | 3-CH₃ | H | H |
| | | | | | | | 4-CHF₂ | | |
| 5-98 | 379.42 | 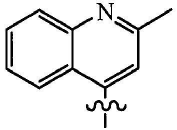 | O | 3-CH₃ | 1 | 2 | 4-H | H | H |
| | | | | | | | 5-CH₃ | | |
| 5-99 | 399.40 | 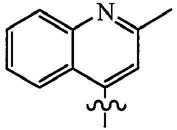 | O | 4-CHF₂ | 1 | 1 | 3-CH₃ | H | H |
| 5-100 | 409.44 | 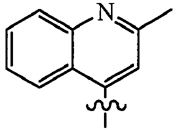 | O | H | 1 | 3 | 2-CH₃ | H | H |
| | | | | | | | 3-CH₃ | | |
| | | | | | | | 4-CH₃ | | |
| 5-101 | 409.44 | 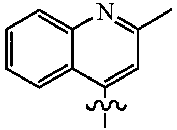 | O | H | 1 | 3 | 2-CH₃ | H | H |
| | | | | | | | 4-CH₃ | | |
| | | | | | | | 5-CH₃ | | |
| 5-102 | 435.42 | 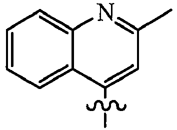 | S | H | 1 | 2 | 3-CF₃ | H | H |
| | | | | | | | 4-H | | |
| 5-103 | 395.48 | 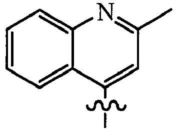 | S | H | 1 | 2 | 3-CH₂CH₃ | H | H |
| | | | | | | | 4-H | | |
| 5-104 | 409.51 | 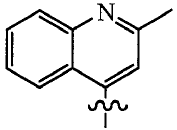 | S | H | 1 | 2 | 3-CH₃ | H | H |
| | | | | | | | 4-CH₂CH₃ | | |
| 5-105 | 409.51 | 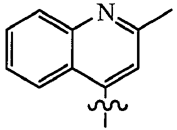 | S | H | 1 | 2 | 3-CH₂CH₃ | H | H |
| | | | | | | | 4-CH₃ | | |
| 5-106 | 447.41 | 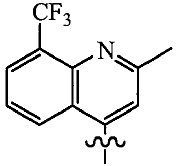 | O | H | 1 | 2 | 3-CH₃ | H | H |
| | | | | | | | 4-CH₃ | | |
| 5-107 | 424.41 | 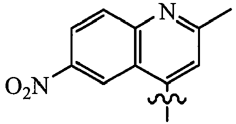 | O | H | 1 | 2 | 3-CH₃ | H | H |
| | | | | | | | 4-CH₃ | | |
| 5-108 | 365.45 | 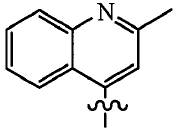 | S | H | 1 | 1 | 3-CH₃ | H | H |
| 5-109 | 395.48 | 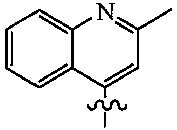 | S | H | 1 | 2 | 3-CH₃ | H | H |
| | | | | | | | 5-CH₃ | | |
| 5-110 | 411.48 | 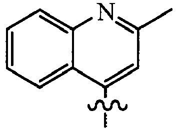 | S | H | 1 | 3 | 3-CH₃ | H | H |
| | | | | | | | 4-H | | |
| | | | | | | | 5-CH₃ | | |
| 5-111 | 431.46 | 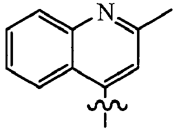 | S | H | 1 | 2 | 3-CHF₂ | H | H |
| | | | | | | | 4-CH₃ | | |
| 5-112 | 437.56 | 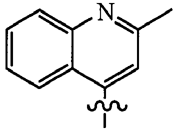 | S | H | 1 | 2 | 3-CH₂CH(CH₃)₂ | H | H |
| | | | | | | | 4-CH₃ | | |
| 5-113 | 449.45 | 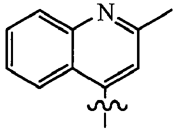 | S | H | 1 | 2 | 3-CF₃ | H | H |
| | | | | | | | 4-H | | |
| 5-114 | 425.51 | 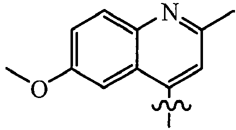 | S | H | 1 | 2 | 3-CH₃ | H | H |
| | | | | | | | 4-CH₃ | | |
| 5-115 | 486.59 | 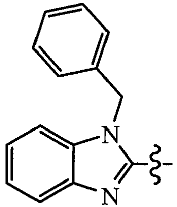 | S | H | 1 | 2 | 3-(2)-propenyl | H | H |
| | | | | | | | 4-CH₃ | | |
| 5-116 | 488.61 | 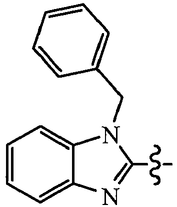 | S | H | 1 | 2 | 3-*n*-propyl | H | H |
| | | | | | | | 4-CH₃ | | |
| 5-117 | 400.46 | 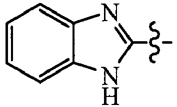 | S | H | 1 | 3 | 3-CH₃ | H | H |
| | | | | | | | 4-CH₃ | | |
| | | | | | | | 5-CH₃ | | |
| 5-118 | 454.50 | 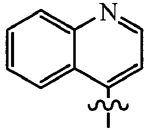 | S | H | 1 | 3 | 3-CH₃ | H | H |
| | | | | | | | 4-CH₂CONH₂ | | |
| | | | | | | | 5-CH₃ | | |
| 5-119 | 447.46 | 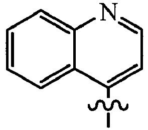 | S | H | 1 | 3 | 3-CH₃ | H | H |
| | | | | | | | 4-CHF₂ | | |
| | | | | | | | 5-CH₃ | | |
| 5-120 | 487.58 | 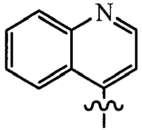 | S | H | 1 | 3 | 3-CH₃ | H | H |
| | | | | | | | 4-benzyl | | |
| | | | | | | | 5-CH₃ | | |
| 5-121 | 399.44 | 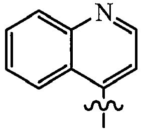 | S | 2-F | 1 | 2 | 4-CH₃ | H | H |
| | | | | | | | 5-CH₃ | | |
| 5-122 | 411.48 | 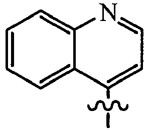 | S | H | 1 | 3 | 3-CH₃ | H | H |
| | | | | | | | 4-CH₃ | | |
| | | | | | | | 5-CH₃ | | |
| 5-123 | 411.48 | 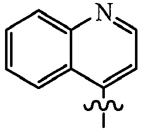 | S | H | 1 | 3 | 2-CH₃ | H | H |
| | | | | | | | 4-CH₃ | | |
| | | | | | | | 5-CH₃ | | |
| 5-124 | 411.48 | 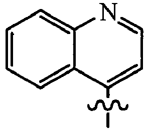 | S | H | 1 | 3 | 2-CH₃ | H | H |
| | | | | | | | 3-CH₃ | | |
| | | | | | | | 4-CH₃ | | |
| 5-125 | 381.45 | 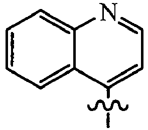 | S | H | 1 | 2 | 3-CH₂CH₃ | H | H |
| | | | | | | | 4-H | | |
| 5-126 | 367.43 | 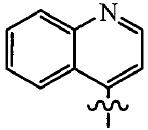 | S | H | 1 | 2 | 3-H | H | H |
| | | | | | | | 4-CH₃ | | |
| 5-127 | 365.41 | 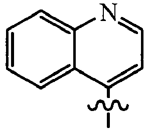 | S | H | 1 | 2 | 3-CH₂-4 | H | H |
| 5-128 | 533.65 | 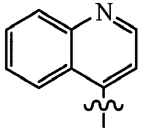 | S | H | 1 | 2 | 3-benzyl | H | H |
| | | | | | | | 4-benzyl | | |
| 5-129 | 525.03 | 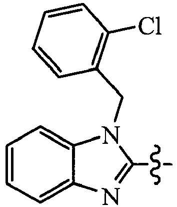 | S | H | 1 | 3 | 3-CH₃ | H | H |
| | | | | | | | 4-CH₃ | | |
| | | | | | | | 5-CH₃ | | |
| 5-130 | 490.57 | 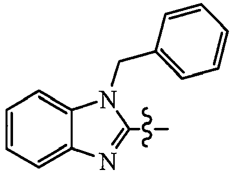 | S | H | 1 | 3 | 3-CH₃ | H | H |
| | | | | | | | 4-CH₃ | | |
| | | | | | | | 5-CH₃ | | |
| 5-131 | 449.45 | 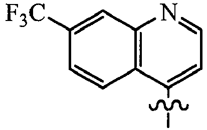 | S | H | 1 | 2 | 3-CH₃ | H | H |
| | | | | | | | 4-CH₃ | | |
| 5-132 | 437.41 | 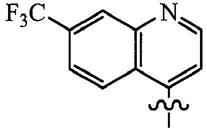 | S | 3-F | 1 | 1 | 4-CH₃ | H | H |
| 5-133 | 437.41 | 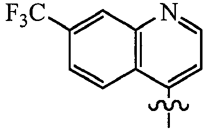 | S | 4-F | 1 | 1 | 3-CH₃ | H | H |
| 5-134 | 453.87 | 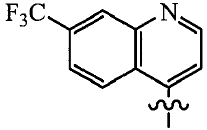 | S | 3-Cl | 1 | 1 | 4-CH₃ | H | H |
| 5-135 | 487.42 | 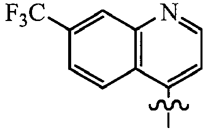 | S | 3-CF₃ | 1 | 1 | 5-CH₃ | H | H |
| 5-136 | 449.45 | 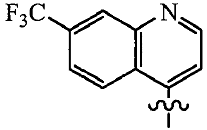 | S | H | 1 | 2 | 3-CH₃ | H | H |
| | | | | | | | 5-CH₃ | | |
| 5-137 | 425.5 | 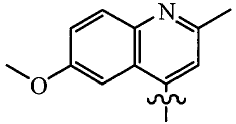 | S | H | 1 | 2 | 3-CH₃ | H | H |
| | | | | | | | 4-CH₃ | | |
| 5-138 | 413.47 | 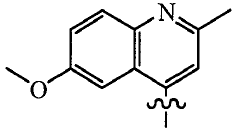 | S | 3-F | 1 | 1 | 4-CH₃ | H | H |
| 5-139 | 413.47 | 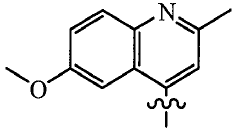 | S | 4-F | 1 | 1 | 3-CH₃ | H | H |
| 5-140 | 429.92 | 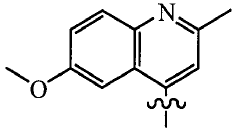 | S | 3-Cl | 1 | 1 | 4-CH₃ | H | H |
| 5-141 | 463.47 | 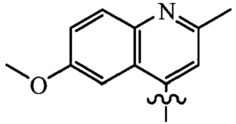 | S | 3-CF₃ | 1 | 1 | 4-CH₃ | H | H |
| 5-142 | 425.5 | 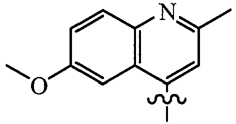 | S | H | 1 | 2 | 3-CH₃ | H | H |
| | | | | | | | 5-CH₃ | | |
| 5-143 | 381.45 | 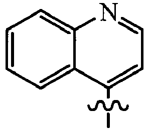 | S | H | 1 | 2 | 3-CH₃ | H | H |
| | | | | | | | 5-CH₃ | | |
| 5-144 | 369.41 | 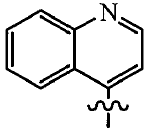 | S | 3-F | 1 | 1 | 4-CH₃ | H | H |
| 5-145 | 369.41 | 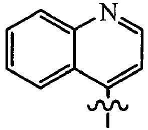 | S | 4-F | 1 | 1 | 3-CH₃ | H | H |
| 5-146 | 385.87 | 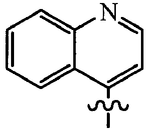 | S | 3-Cl | 1 | 1 | 4-CH₃ | H | H |
| 5-147 | 419.42 | 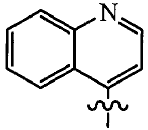 | S | 3-CF₃ | 1 | 1 | 4-CH₃ | H | H |
| 5-148 | 381.45 | 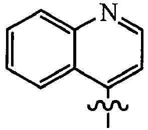 | S | H | 1 | 2 | 3-CH₃ | H | H |
| | | | | | | | 5-CH₃ | | |
| 5-149 | 395.47 | 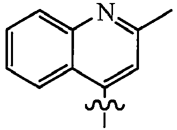 | S | H | 1 | 2 | 3-CH₃ | H | H |
| | | | | | | | 4-CH₃ | | |
| 5-150 | 383.44 | 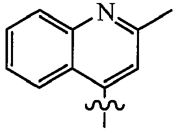 | S | 3-F | 1 | 1 | 4-CH₃ | H | H |
| 5-151 | 383.44 | 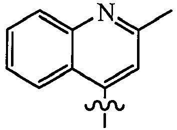 | S | 4-F | 1 | 1 | 3-CH₃ | H | H |
| 5-152 | 399.89 | 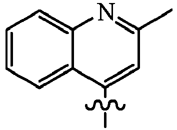 | S | 3-Cl | 1 | 1 | 4-CH₃ | H | H |
| 5-153 | 433.45 | 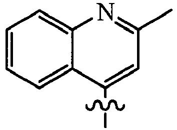 | S | 3-CF3 | 1 | 1 | 4-CH₃ | H | H |
| 5-154 | 395.47 | 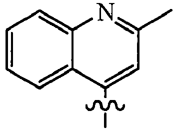 | S | H | 1 | 2 | 3-CH₃ | H | H |
| | | | | | | | 5-CH₃ | | |
| 5-155 | 474.37 | 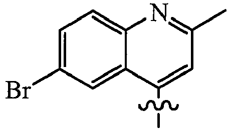 | S | H | 1 | 2 | 3-CH₃ | H | H |
| | | | | | | | 4-CH₃ | | |
| 5-156 | 462.34 | 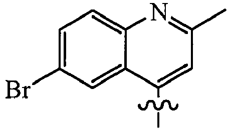 | S | 3-F | 1 | 1 | 4-CH₃ | H | H |
| 5-157 | 462.34 | 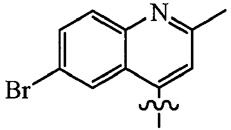 | S | 4-F | 1 | 1 | 3-CH₃ | H | H |
| 5-158 | 478.79 | 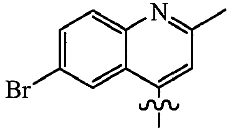 | S | 3-Cl | 1 | 1 | 4-CH₃ | H | H |
| 5-159 | 512.34 | 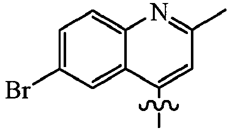 | S | 3-CF₃ | 1 | 1 | 5-CH₃ | H | H |
| 5-160 | 474.37 | 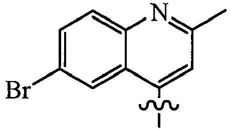 | S | H | 1 | 2 | 3-CH₃ | H | H |
| | | | | | | | 5-CH₃ | | |
| 5-161 | 419.50 | 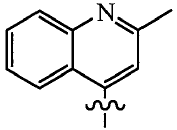 | S | R¹ and R² taken together (*see* R₂) | 1 | 2 | 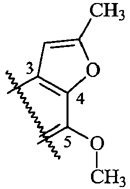 | H | H |
| 5-162 | 440.52 | 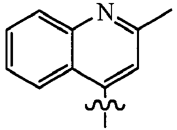 | S | H | 1 | 3 | 3-CH₃ | CH 3 | H |
| | | | | | | | 4-CH₃ | | |
| | | | | | | | 5-CH₃ | | |
| 5-163 | 435.55 | 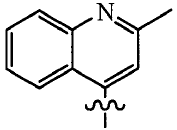 | S | H | 1 | 2 | 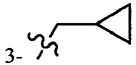 | H | H |
| | | | | | | | 4-CH₃ | | |
| 5-164 | 441.50 | 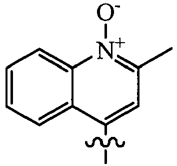 | S | H | 1 | 3 | 3-CH₃ | H | H |
| | | | | | | | 4-CH₃ | | |
| | | | | | | | 5-CH₃ | | |
| 5-165 | 439.53 | 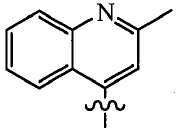 | S | H | 2 | 3 | 3-CH₃ | H | H |
| | | | | | | | 4-CH₃ | | |
| | | | | | | | 5-CH₃ | | |
| 5-166 | 422.48 | 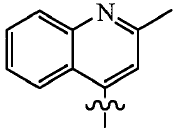 | NR⁶ (R⁶= CH₃) | H | 1 | 3 | 3-CH₃ | H | H |
| | | | | | | | 4-CH₃ | | |
| | | | | | | | 5-CH₃ | | |
| 5-167 | 454.54 | 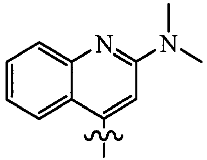 | S | H | 1 | 3 | 3-CH₃ | H | H |
| | | | | | | | 4-CH₃ | | |
| | | | | | | | 5-CH₃ | | |
| 5-168 | 361.42 | 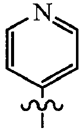 | S | H | 1 | 3 | 3-CH₃ | H | H |
| | | | | | | | 4-CH₃ | | |
| | | | | | | | 5-CH₃ | | |
| 5-169 | 362.41 | 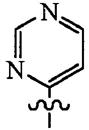 | S | H | 1 | 3 | 3-CH₃ | H | H |
| | | | | | | | 4-CH₃ | | |
| | | | | | | | 5-CH₃ | | |
| 5-170 | 467.44 | 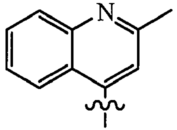 | S | H | 1 | 2 | 3-CHF₂ | H | H |
| | | | | | | | 4-CHF₂ | | |
| 5-171 | 387.40 | 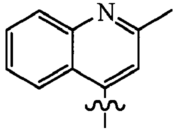 | S | 3-F | 1 | 1 | 4-H | H | H |
| | | | | 5-F | | | | | |
| 5-172 | 438.54 | 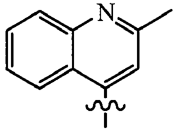 | S | 3-CH₂N (CH₃)₂ | 1 | 1 | 4-H | H | H |
| 5-173 | 401.43 | 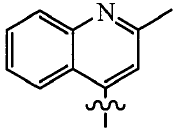 | S | 3-F | 1 | 1 | 4-CH₃ | H | H |
| | | | | 5-F | | | | | |
| 5-174 | 385.37 | 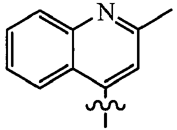 | O | 2-F | 1 | 1 | 3-CH₃ | H | H |
| | | | | 4-F | | | | | |
| 5-175 | 431.46 | 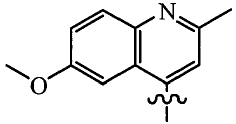 | S | 3-F | 1 | 1 | 4-CH₃ | H | H |
| | | | | 5-F | | | | | |
| 5-176 | 431.46 | 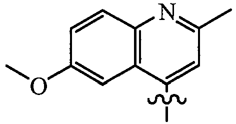 | S | 2-F | 1 | 1 | 4-CH₃ | H | H |
| | | | | 3-F | | | | | |
| 5-177 | 401.43 | 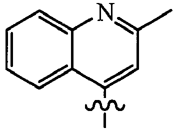 | S | 2-F | 1 | 1 | 4-CH₃ | H | H |
| | | | | 3-F | | | | | |
| 5-178 | 401.43 | 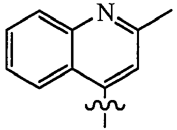 | S | 3-F | 1 | 1 | 4-CH₃ | H | H |
| | | | | 5-F | | | | | |
| 5-179 | 387.40 | 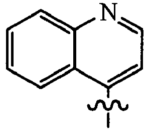 | S | 3-F | 1 | 1 | 4-CH₃ | H | H |
| | | | | 5-F | | | | | |
| 5-180 | 381.45 | 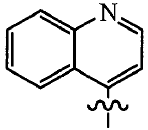 | S | H | 1 | 2 | 3-CH₃ | H | H |
| | | | | | | | 4-CH₃ | | |
| 5-181 | 455.40 | 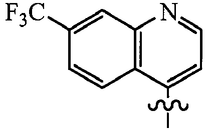 | S | 2-F | 1 | 1 | 4-CH₃ | H | H |
| | | | | 3-F | | | | | |
| 5-182 | 455.40 | 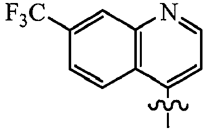 | S | 3-F | 1 | 1 | 4-CH₃ | H | H |
| | | | | 5-F | | | | | |

### EXAMPLE 6

### (E)-3-(1-BENZYL-1H-BENZO[D]IMIDAZOL-2-YL)-N'-(3,4-DIMETHOXYBENZYLIDENE)PROPANEHYDRAZIDE

### Step 6A Preparation of ethyl 3-(1H-benzo[d]imidazol-2-yl)propanoate 6a

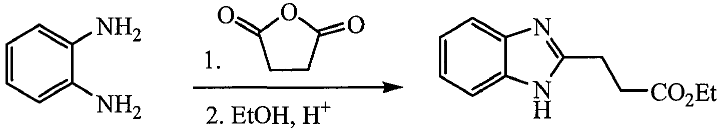

To a stirred solution of benzene-1,2-diamine (20.0 g, 0.19 mol, 1.0 eq.) in dioxane (500 mL) at room temperature, was added succinic anhydride (22.2 g, 0.22 mol, 1.2 eq.), and the reaction mixture was heated to 80 °C and stirred at that temperature for 16 hours. The resulting precipitate was filtered and washed with dioxane (100 mL), water (100 mL) and diethyl ether (100 mL) to give a white solid. The solid was suspended in ethanol (400 mL), and concentrated sulfuric acid (10 mL) was added at room temperature. The reaction mixture was heated to reflux and stirred at that temperature for 20 hours. The reaction was then cooled to room temperature and concentrated *in vacuo.* Water (100 mL) was added, and the mixture was adjusted to pH 7 with 1 M aqueous NaOH solution. The aqueous layer was extracted into ethyl acetate (3 x 100 mL), and the combined organic extracts were dried over magnesium sulfate and concentrated *in vacuo* to give **6a** as a pale-orange oil (15.6 g, 46%).

### Step 6B Preparation of ethyl 3-(1-benzyl-1H-benzo[d]imidazol-2-yl)propanoate 6b

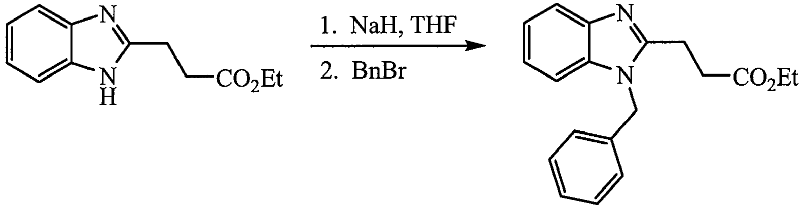

To a stirred solution of ethyl 3-(1*H*-benzo[*d*]imidazol-2-yl)propanoate **6a** (2.85 g, 13.1 mmol, 1.0 eq.) in tetrahydrofuran (100 mL) at 0 °C, was added 60% sodium hydride in mineral oil (784 mg, 19.6 mmol, 1.5 eq.), and the reaction was stirred at 0 °C for 20 minutes. Benzyl bromide (4.47 g, 26.1 mmol, 2.0 eq.) was added at 0 °C, and the reaction was stirred, with warming to room temperature, for 16 hours. The mixture was concentrated *in vacuo* and water (100 mL) was carefully added. The aqueous layer was extracted with dichloromethane (3 x 75 mL), and the combined organic extracts were washed with 1M hydrochloric acid (100 mL) and brine (100 mL). The organic layer was dried over magnesium sulfate and concentrated *in vacuo.* Purification by flash chromatography (5% MeOH:95% EtOAc) gave **6b** as an orange oil (3.32 g, 82%).

### Step 6C Preparation of 3-(1-benzyl-1H-benzo[d]imidazol-2-yl)propanehydrazide 6c

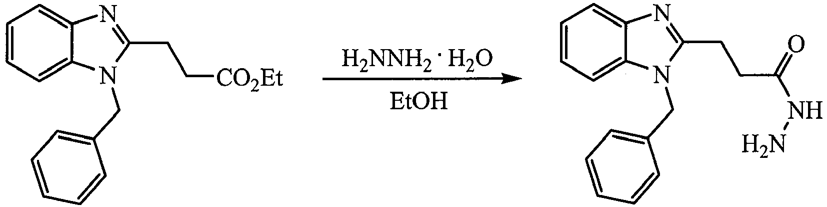

Step 6C was performed in an analogous fashion to Example 4, Step 4C.

### Step 6D Preparation of (E)-3-(1-benzyl-1H-benzo[d]imidazol-2-yl)-N'-(3,4-dimethoxybenzylidene)propanehydrazide 6-1

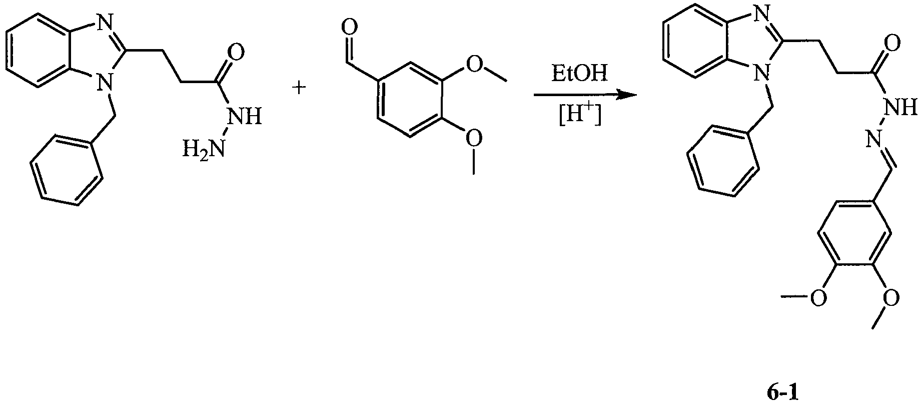

Step 6D was prepared in an analogous fashion to Example 4, Step 4D.

Referring to Table 4, the following derivatives of structure (V), wherein R, R³, R⁴ and R⁵ are hydrogen, were synthesized according to the procedures outlined in Example 6.

**Table 4**

| |
|---|
| 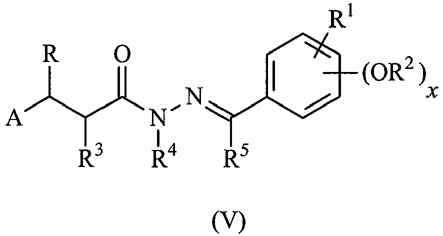 |

| No. | MW | A | R¹ | *x* | R² |
|---|---|---|---|---|---|
| 6-1 | 442.52 | 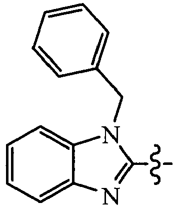 | H | 2 | 3-CH₃ |
| | | | | | 4-CH₃ |
| 6-2 | 382.42 | 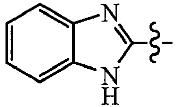 | H | 2 | 3-CH₃ |
| | | | | | 4-CH₃ |
| | | | | | 5-CH₃ |
| 6-3 | 387.44 | 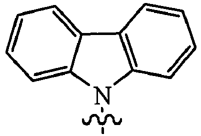 | H | 2 | 3-CH₃ |
| | | | | | 4-H |
| 6-4 | 320.39 | 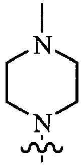 | H | 2 | 3-H |
| | | | | | 4-CH₃ |
| 6-5 | 336.35 | 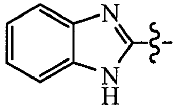 | H | 2 | 3-CH₂-4 |
| 6-6 | 338.37 | 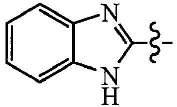 | H | 2 | 3-H |
| | | | | | 4-CH₃ |
| 6-7 | 352.39 | 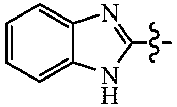 | H | 2 | 3-CH₂CH₃ |
| | | | | | 4-H |
| 6-8 | 382.42 | 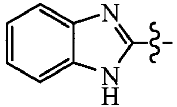 | H | 3 | 2-CH₃ |
| | | | | | 3-CH₃ |
| | | | | | 4-CH₃ |

| No. | MW | A | R¹ | *x* | R² |
|---|---|---|---|---|---|
| 6-9 | 382.42 | 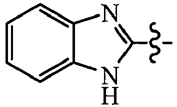 | H | 3 | 2-CH₃ |
| | | | | | 4-CH₃ |
| | | | | | 5-CH₃ |
| 6-10 | 370.38 | 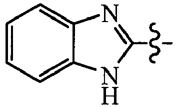 | 2-F | 2 | 4-CH₃ |
| | | | | | 5-CH₃ |
| 6-11 | 458.52 | 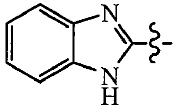 | H | 3 | 3-CH₃ |
| | | | | | 4-benzyl |
| | | | | | 5-CH₃ |
| 6-12 | 456.55 | 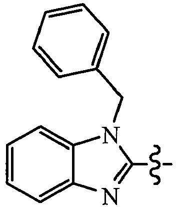 | H | 2 | 3-CH₃ |
| | | | | | 4-CH₂CH₃ |
| 6-13 | 456.55 | 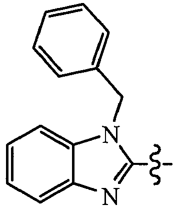 | H | 2 | 3-CH₂CH₃ |
| | | | | | 4-CH₃ |

### EXAMPLE 7

Referring to Table 5, the following derivatives of structure (IV), wherein R⁴ and R⁵ are hydrogen, can be synthesized according to Reaction Scheme 5.

**Table 5**

| | | | | | |
|---|---|---|---|---|---|
| 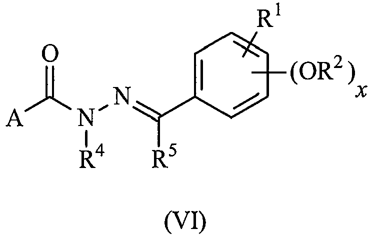 | | | | | |

| No. | MW | A | R¹ | *x* | R² |
|---|---|---|---|---|---|
| 7-1 | 304.30 | 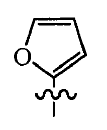 | H | 3 | 3-CH₃ |
| | | | | | 4-CH₃ |
| | | | | | 5-CH₃ |
| 7-2 | 380.40 | 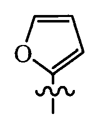 | H | 2 | 3-CH₃ |
| | | | | | 4-benzyl |
| | | | | | 5-CH₃ |
| 7-3 | 272.26 | 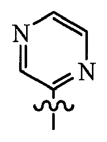 | H | 2 | 3-CH₃ |
| | | | | | 4-H |
| 7-4 | 271.28 | 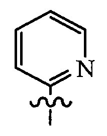 | H | 2 | 3-H |
| | | | | | 4-CH₃ |
| 7-5 | 323.35 | 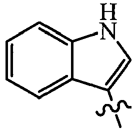 | H | 2 | 3-CH₂CH₃ |
| | | | | | 4-H |
| 7-6 | 328.32 | 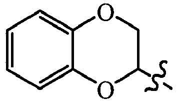 | H | 2 | 3-CH₃ |
| | | | | | 4-H |
| 7-7 | 328.32 | 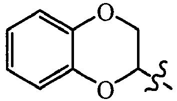 | H | 2 | 3-H |
| | | | | | 4-CH₃ |
| 7-8 | 258.23 | 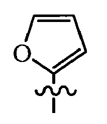 | H | 2 | 3-CH₂-4 |
| 7-9 | 260.25 | 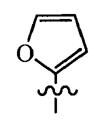 | H | 2 | 3-H |
| | | | | | 4-CH₃ |
| 7-10 | 274.28 | 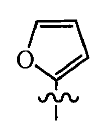 | H | 2 | 3-CH₂CH₃ |
| | | | | | 4-H |
| 7-11 | 304.30 | 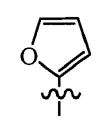 | H | 3 | 2-CH₃ |
| | | | | | 3-CH₃ |
| | | | | | 4-CH₃ |
| 7-12 | 304.30 | 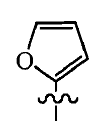 | H | 3 | 2-CH₃ |
| | | | | | 4-CH₃ |
| | | | | | 5-CH₃ |
| 7-13 | 292.27 | 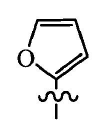 | 2-F | 2 | 4-CH₃ |
| | | | | | 5-CH₃ |
| 7-14 | 340.28 | 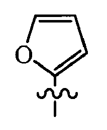 | H | 3 | 3-CH₃ |
| | | | | | 4-CHF₂ |
| | | | | | 5-CH₃ |
| 7-15 | 347.33 | 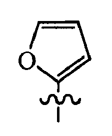 | H | 3 | 3-CH₃ |
| | | | | | 4-CH₂CONH₂ |
| | | | | | 5-CH₃ |

### EXAMPLE 8

### Compound Assay

### PDE10 Biochemical Assay

The phosphodiesterase (PDE) assay was performed using recombinant human PDE 1A3, 2A3, 3 catalytic region, 4 catalytic region, 5 catalytic region, 7A, 8A, 9A2, 10A1 and 11A1 enzymes expressed in a baculoviral system using Sf9 cells. PDE activity was measured using a modification of the two-step method of Thompson and Appleman described above which was adapted for 96 well plate format. The effect of the PDE inhibitors was determined by assaying a fixed amount of the enzyme in the presence of test compound concentrations and a substrate concentration below that of the Km, so that Ki equals IC₅₀. The final assay volume was 110 µl with assay buffer (10mM MgCl₂; 40mM Tris.HCl; pH 7.4). Reactions were initiated with enzyme and incubated with (³H) -substrate and substance for 20 minutes at 30° C. The reaction was terminated by denaturing the enzyme (heating the reaction to 70° C for 2 minutes). The reaction was then cooled at 4° C for 10 minutes before the addition of snake venom (*Crotalus atrox,* 0.2 mg/ml) for 10 minutes at 30° C, thus allowing non-specific hydrolysis of the tritiated substrate. Separation of the remaining unhydrolysed cyclic nucleotide was achieved by a batch binding of the mixture to activated Dowex (200 µl) anion exchange resin. The anion exchange resin bound the charged nucleotides, leaving only hydrolysed (³H) substrate in the soluble fraction. The soluble fraction (50 µl) was then added to microscint-20 (200 µl) and counted on a Top Count Plate reader. Radioactivity units were plotted against inhibitor concentration and IC₅₀ values obtained using Graph Pad Prism software.

In the above assay, compounds of this invention are PDE10 inhibitors with an IC₅₀ of 100µM or less, generally less than 10 µM, and typically less than 1 µM. To this end, compounds 1-1, 2-2, 4-1, 5-1, 5-2, 5-3, 5-4, 5-5, 5-25, 5-26, 5-27, 5-64, 5-67, 5-73, 5-75, 5-76, 5-77, 5-79, 5-91, 5-92, 5-104, 5-105, 5-108, 5-109, 5-110, 5-111, 5-112, 5-114, 5-115, 5-118, 5-119, 5-121, 5-122, 5-123, 5-125, 5-129, 5-130, 5-161, 5-162 and 5-163 were found to have IC₅₀'s of less than or equal to 1 µM.

### EXAMPLES 9-14

### Evaluation of Representative Compounds in Behavioral Models

Schizophrenia has been associated with dysfunctions of dopaminergic, glutamatergic and serotonergic neurotransmission. Psychostimulant drugs in these three classes, dopaminergic agonists (such as amphetamine and apomorphine), glutamatergic antagonists (such as PCP and ketamine), and serotonergic agonists (such as LSD and MDMA), all induce psychotomimetic states (*e.g.,* hyperactivity and disruption of prepulse inhibition) in animals that closely resemble schizophrenia symptoms in humans. Known antipsychotic drugs, including both typical antipsychotics (*e.g.,* haloperidol) and atypical antipsychotics (*e.g.,* olanzapine), reverse such psychotomimetic states in animals. Examples 9-14 described below evaluate representative compounds of the present invention in animal behavioral models to compare the resulting effect to that of known antipsychotics. Methods used in the Examples 9-14 are as follows.

Prepulse inhibition (PPI) of the acoustic startle response evaluates the brain's sensorimotor gating function that is often disrupted in schizophrenic and other psychotic conditions. Psychostimulants such as PCP and amphetamine reduce or disrupt PPI, and many antipsychotics increase PPI and/or reverse psychostimulant-induced reduction of PPI. In addition, the startle response to the loud noise itself (in the absense of any prepulse) is a measure of the basic sensorimotor reflex. The test is done using the SR-Lab System (San Diego Instruments, San Diego, CA). A test session consists of six trial types under the background noise of 70 dB. One type uses a 40 msec, 120 dB noise as the startle stimulus. Four types contain acoustic startle stimulus preceded by acoustic prepulses of different intensity: the 20-msec prepulse noise of 73, 76, 79, or 82 dB is presented 100 msec before the 120 dB startle stimulus. The last trial type uses the 70 dB background noise with no startle stimulus to measure baseline reaction. Six blocks of the six trial types are presented in pseudorandom order. The startle response is recorded for 65 ms starting with the onset of the startle stimulus. Measurements used to assess PPI are the maximum startle amplitude and the percent each of the 4 prepulses inhibits the startle response.

Psychostimulant-induced hyperactivity is measured by injecting animals with PCP or amphetamine and monitoring the animals' activity levels in the VersaMax chambers (Accuscan Instruments, Columbus, OH) measuring 40 x 40 cm. Locomotor activity is detected by photobeam breaks as the animal crosses each beam. The animal is placed in the center of the field and left undisturbed for a period of time (20 min to 2 hr) to measure its spontaneous activity in a novel environment. Measurements used to assess locomotor activity include: horizontal activity, total distance traveled, vertical activity (rearing events - animal raises up on hindlimbs), rotation, stereotypy, and distance traveled in the center compared to total distance traveled (center: total distance ratio). Psychostimulants, including dopamine agonist amphetamine and NMDA antagonist PCP, induce psychosis-like conditions manifested as hyperactivity and increased stereotypic behavior. Known antipsychotics are able to reverse psychostimulant-induced hyperactivity and increased stereotypy.

Conditioned avoidance response (CAR) is a behavioral test to evaluate antipsychotic effect of a test compound. It utilizes a shuttle box (Med Associates, St. Albans, VT) with two equal chambers separated by a retractable door. Each chamber is fitted with metal grid floor that is capable of delivering electric shocks independently. A computer program is used to implement the testing paradigm as well as record the animal's movement between the two chambers through infrared beam sensors. The testing paradigm is as the follows. A mouse is placed into one chamber. A light (conditioned stimulus, CS) comes on. Five seconds later, mild electric shocks (0.4 mA) (unconditioned stimulus, US) are delivered to the chamber where the mouse is located (as detected by infrared beams) until the mouse escapes to the adjacent chamber or until 10 sec has elapsed. The US and CS always co-terminate. With randomized inter-trial intervals averaging 15 sec, 30 such CS-US pairing trials are given to each mouse each day. For each trial, an escape response is registered if the mouse crosses to the other chamber after being shocked (*i.e.,* during the 10-sec US period), and an avoidance response is registered if the mouse crosses to the other chamber during the first 5-sec CS only period. The animals are trained in such paradigm for 15-20 days, during which the average percentage of avoidance responses will improve to 60-80%. This indicates that animals have learned to avoid the onset of footshocks by moving to the opposite chamber upon activation of the CS (light). These trained animals are then used for compound testing using the same paradigm. Known antipsychotics have been found to inhibit the conditioned avoidance response, and the ability of new compounds to inhibit this response is thought to be predictive of antipsychotic effect in humans.

### EXAMPLE 9

### Increased Prepulse Inhibition for Compound 5-1

Compound 5-1 (Table 3) was found to increase prepulse inhibition (PPI) similar to olanzapine, as shown in FIGURE 1. C57BL/6 male mice were injected intraperitoneally (i.p.) with either compound or vehicle. Thirty minutes after injection, the mice were transferred to the PPI testing chamber and evaluated. FIGURE 1A shows that olanzapine (3 mg/kg) significantly decreases the startle response (left panel) and increases PPI at 3 different prepulse levels (right panel) compared to vehicle control (* p<0.05, n=8 per group, student's t-test). FIGURE 1B shows that compound 5-1 (50 mg/kg) does not affect the startle response (left panel) but significantly increases PPI at 3 different prepulse levels (right panel) compared to vehicle control (* p<0.05, ** p<0.01, n=24 per group, student's t-test).

### EXAMPLE 10

### Reduction of PCP-Induced Hyperactivity for Compound 5-1

Compound 5-1 (Table 3) was found to reduce PCP-induced hyperactivity similar to olanzapine and haloperidol, as shown in FIGURE 2. C57BL/6 male mice were injected with either compound or vehicle via i.p. Ten minutes later, the mice were injected with PCP (5 mg/kg) (or vehicle as in FIGURE 2B) via i.p. The mice were placed in the activity chambers 10 minutes after PCP injection and their locomotor activities were monitored by infrared beam breaks for 20 min. FIGURE 2A shows that both olanzapine (0.2 mg/kg) and haloperidol (0.2 mg/kg) significantly reduce the hyperactivity (left panel) and stereotypy (right panel) induced by PCP as seen in the vehicle+PCP control (p<0.001, n=8 per group, repeated measures ANOVA). FIGURE 2B shows that compound 5-1 (50 mg/kg) completely abolishes the hyperactivity (left panel) and stereotypy (right panel) induced by PCP as seen in the vehicle+PCP control (p<0.001, n=8 per group, repeated measures ANOVA).

### EXAMPLE 11

### Reduction of Amphetamine-Induced Hyperactivity for Compound 5-1

Compound 5-1 (Table 3) was found to reduce amphetamine-induced hyperactivity similar to olanzapine, as shown in FIGURE 3. C57BL/6 male mice were injected with either compound or vehicle via i.p. Ten minutes later, the mice were injected with amphetamine (2 mg/kg in (FIGURE 3A) or 5 mg/kg in (FIGURE 3B)) via i.p. The mice were placed in the activity chambers 10 minutes after amphetamine injection and their locomotor activities were monitored by infrared beam breaks for 20 min. FIGURE 3A shows that olanzapine (0.2 mg/kg) partially but significantly reduce the hyperactivity (left panel) and stereotypy (right panel) induced by amphetamine ("amph") as seen in the vehicle+amph control (p<0.05, n=8 per group, repeated measures ANOVA). FIGURE 3B shows that compound 5-1 (50 mg/kg) also partially but significantly reduce the hyperactivity (left panel) and stereotypy (right panel) induced by amphetamine as seen in the vehicle+amph control (p<0.01, n=8 per group, repeated measures ANOVA).

### EXAMPLE 12

### Reduction of Conditioned Avoidance Response for Compound 5-1

Compound 5-1 (Table 3) was found to reduce Conditioned Avoidance Response (CAR) similar to haloperidol and olanzapine, as shown in FIGURE 4. C57BL/6 male mice were trained in the CAR paradigm to predict and avoid the noxious stimulus, reaching a plateau of approximately 20-25 avoidance responses per 30 trials ("training plateau") each day. The mice were then injected with compound or vehicle via i.p., and 20 minutes later they were tested for 30 trials in the CAR paradigm. Vehicle treatment and compound treatment were given to the same animals on alternating days, and the effect of compound in reducing avoidance response was analyzed through within-subject comparison (paired t-test). Vehicle exposure ("vehicle") does not alter the avoidance response of these trained animals. FIGURE 4A shows that haloperidol (0.15 mg/kg) significantly reduces the number of avoidance response (*** p<0.001, n=6 per group, paired t-test). FIGURE 4B shows that olanzapine (0.45 mg/kg) significantly reduces the number of avoidance response (** p<0.01, n=6 per group, paired t-test). FIGURE 4C shows that compound 5-1 (30 mg/kg) significantly reduces the number of avoidance response (*** p<0.001, n=6 per group, paired t-test). In all these cases, the numbers of escape response increase correspondingly and the total numbers of transitions between the two chambers do not change (data not shown), indicating a specific reduction of CAR that is not due to compromised motor function.

### EXAMPLE 13

### Reduction of Conditioned Avoidance Response and Hyperactivity for Compound 5-110

Compound 5-110 (Table 3) was found to exhibit antipsychotic properties in two behavioral tests, as shown in FIGURE 5. FIGURE 5A shows that compound 5-110 (10 mg/kg) significantly reduces the number of avoidance response in the CAR test (** p<0.01, n=6 per group, paired t-test). Experimental procedure was the same as in Example 12. FIGURE 5B shows that compound 5-110 (30 mg/kg) significantly reduces the hyperactivity (left panel) and stereotypy (right panel) induced by PCP (5 mg/kg) (p<0.001, n=8 per group, repeated measures ANOVA). Experimental procedure was the same as in Example 10.

### EXAMPLE 14

### Reduction of Conditioned Avoidance Response for Compound 5-103

Compound 5-103 (Table 3) was found to reduce Conditioned Avoidance Response (CAR), as shown in FIGURE 6. Compound 5-103 (10 mg/kg) significantly reduces the number of avoidance response (** p<0.01, n=6 per group, paired t-test). Experimental procedure was the same as in Example 12.

## Claims

1. A compound for use in treating schizophrenia in a warm-blooded animal in need thereof, said compound having the following structure (I): 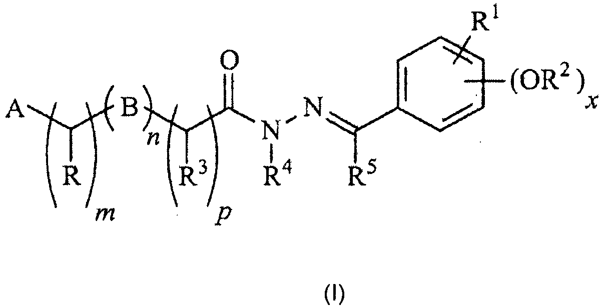 or a pharmaceutically acceptable salt, stereoisomer, or solvate thereof,
wherein:
*m*, *n* and *p* are individually 0 or 1;
*x* is 1, 2 or 3;
A is an optionally substituted heterocycle;
B is -O-, -NR⁶- or -S(O)_{z}- where z is 0, 1 or 2;
R is hydrogen or oxo;
R¹ is absent or represents 1, 2 or 3 substituents that are the same or different and independently halogen, C₁₋₆alkyl, -CHF₂, -CF₃, -CH₂NH₂, -CH₂NH(C₁₋₆alkyl) or -CH₂N(C₁₋₆alkyl)₂;
R² is at each occurrence the same or different and independently hydrogen, C₁₋₆alkyl, -C(=O)(C₁₋₆alkyl), benzyl, -CH₂CONH₂, -CHF₂, -CF₃, or any two R² groups may be taken together to form a C₁₋₆alkanediyl; and
R³, R⁴, R⁵ and R⁶ are the same or different and independently hydrogen or C₁₋₆alkyl.

2. Use of a compound for the preparation of a medicament for use in treating schizophrenia in a warm-blooded animal in need thereof, said compound having the following structure (I): 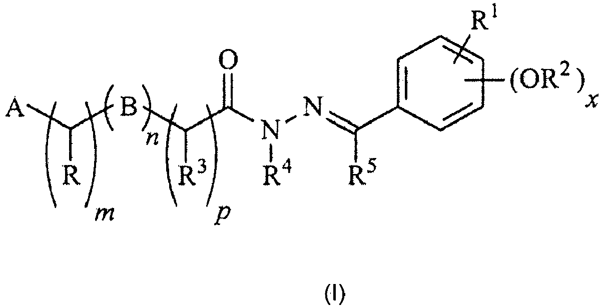 or a pharmaceutically acceptable salt, stereoisomer, or solvate thereof,
wherein:
*m*, *n* and *p* are individually 0 or 1;
*x* is 1, 2 or 3;
A is an optionally substituted heterocycle;
B is -O-, -NR⁶- or -S(O)_{z}- where z is 0, 1 or 2;
R is hydrogen or oxo;
R¹ is absent or represents 1, 2 or 3 substituents that are the same or different and independently halogen, C₁₋₆alkyl, -CHF₂, -CF₃, -CH₂NH₂, -CH₂NH(C₁₋₆alkyl) or -CH₂N(C₁₋₆alkyl)₂;
R² is at each occurrence the same or different and independently hydrogen, C₁₋₆alkyl, -C(=O)(C₁₋₆alkyl), benzyl, -CH₂CONH₂, -CHF₂, -CF₃, or any two R² groups may be taken together to form a C₁₋₆alkanediyl; and
R³, R⁴, R⁵ and R⁶ are the same or different and independently hydrogen or C₁₋₆alkyl.

3. The compound for use or use according to claim 1 or 2, wherein the compound has the following structure (II):

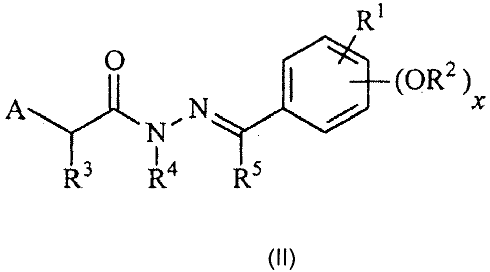

4. The compound for use or use according to claim 1 or 2, wherein the compound has the following structure (III):

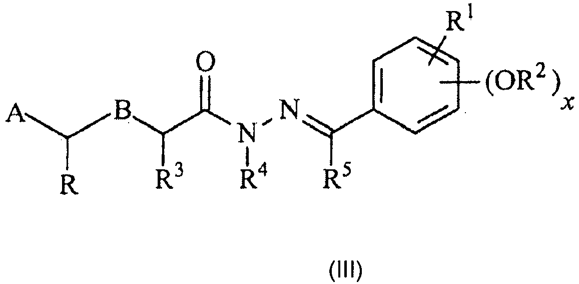

5. The compound for use or use according to claim 1 or 2, wherein the compound has the following structure (IV):

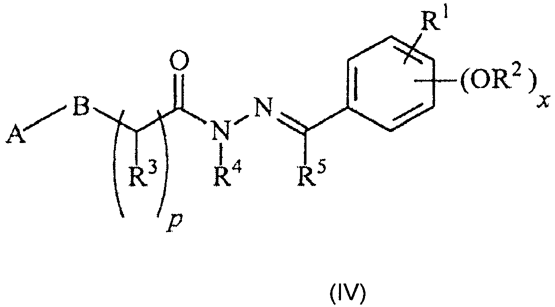

6. The compound for use or use according to claim 1 or 2, wherein the compound has the following structure (V):

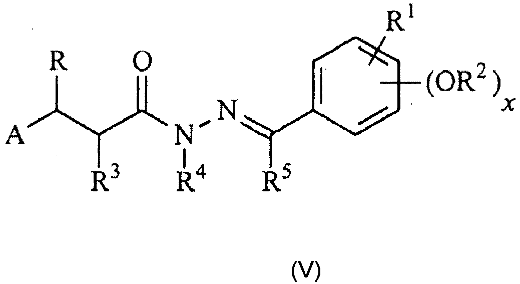

7. The compound for use or use according to claim 1 or 2, wherein the compound has the following structure (VI):

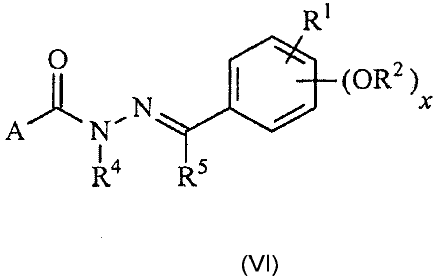

8. The compound for use or use according to claim 1 or 2, wherein the compound has the following structure (IVa):

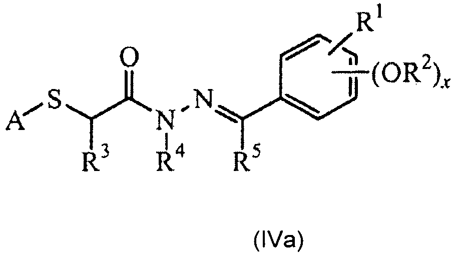

9. The compound for use or use according to claim 1 or 2, wherein the compound has the following structure (IVb):

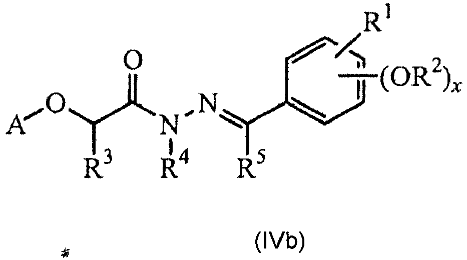

10. The compound for use or use according to claim 1 or 2, wherein the compound has the following structure (IVc):

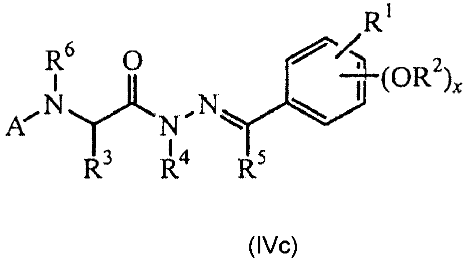

11. The compound for use or use according to claim 1 or 2, wherein the compound has the following structure (IVg): 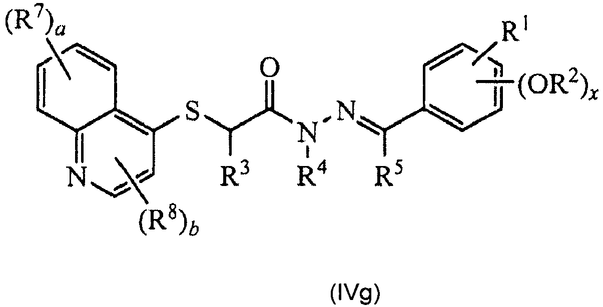 wherein:
*a* is 1, 2, 3 or 4;
*b* is 1 or 2; and
R⁷ and R⁸ are the same or different and independently hydrogen, halogen, C₁₋₆alkyl, -O(C₁₋₆alkyl), C₁₋₆haloalkyl or nitro.

12. The compound for use or use according to claim 1 or 2, wherein the compound has the following structure (IVh): 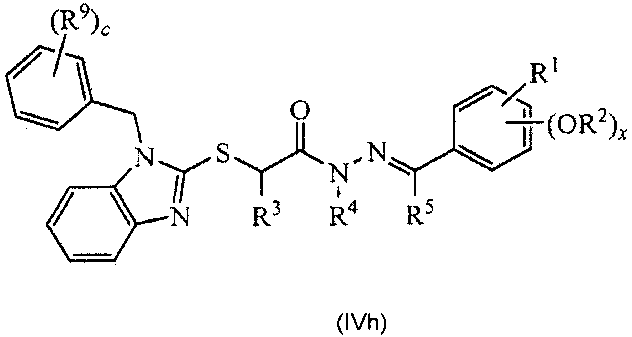 wherein:
c is 1, 2, 3, 4 or 5; and
R⁹ is at each occurrence the same or different and independently, hydrogen, halogen or C₁₋₆alkyl.

13. A compound having the following structure (IVg): 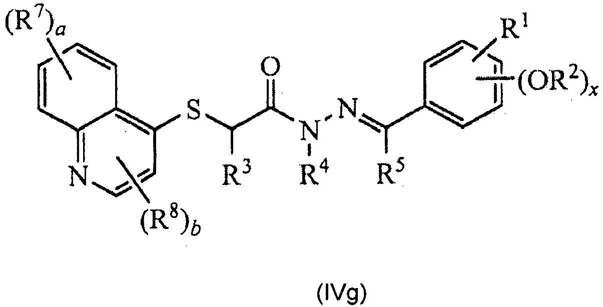 or a pharmaceutically acceptable salt, stereoisomer, or solvate thereof,
wherein:
*a* is 1, 2, 3 or 4;
*b* is 1 or 2;
*x* is 1, 2 or 3 with the proviso that when *x* is 1, R¹ is not a single methyl substituent;
R¹ represents 1 or 2 substitutents independently halogen, C₁₋₆alkyl, -CHF₂ or -CF₃;
R² is at each occurrence the same or different and independently, hydrogen, C₁₋₆alkyl, benzyl, -CH₂CONH₂, -CHF₂, -CF₃, or any two R² groups may be taken together to form a C₁₋₆alkanediyl; and
R³, R⁴ and R⁵ are the same or different and independently hydrogen or C₁₋₆alkyl; and
R⁷ and R⁸ are the same or different and independently hydrogen, halogen, C₁₋₆alkyl, -O(C₁₋₆alkyl), C₁₋₆haloalkyl or nitro.

14. The compound of claim 13 wherein R³, R⁴ and R⁵ are hydrogen.

15. A pharmaceutical composition comprising a compound of claim 13 and a pharmaceutically acceptable carrier or diluent.

## Patentansprüche

1. Verbindung zur Verwendung beim Behandeln von Schizophrenie in einem warmblütigen Lebewesen, das dieses benötigt, wobei die Verbindung die folgende Struktur (I) hat: 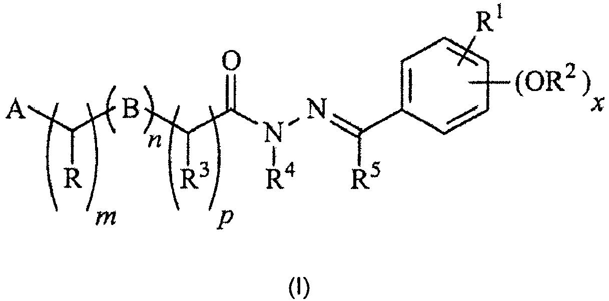 oder ein pharmazeutisch verträgliches Salz, Stereoisomer oder Solvat davon,
wobei:
*m, n* und *p* individuell 0 oder 1 sind;
*x* 1, 2 oder 3 ist;
A ein gegebenenfalls substituierter Heterocyclus ist;
B -O-, -NR⁶- oder -S(O)_{z}- ist, wobei *z* 0, 1 oder 2 ist;
R Wasserstoff oder Oxo ist;
R¹ abwesend ist oder 1, 2 oder 3 Substituenten bedeutet, welche gleich oder unterschiedlich sind und unabhängig Halogen, C₁₋₆-Alkyl, -CHF₂, -CF₃, -CH₂NH₂, -CH₂NH(C₁₋₆-Alkyl) oder -CH₂N(C₁₋₆-Alkyl)₂ sind;
R² bei jedem Vorkommen gleich oder unterschiedlich ist und unabhängig Wasserstoff, C₁₋₆-Alkyl, -C(=O)(C₁₋₆-Alkyl), Benzyl, -CH₂CONH₂, -CHF₂, -CF₃ ist, oder beliebige zwei R²-Gruppen zusammengefasst werden können, um ein C₁₋₆-Alkandiyl zu bilden; und
R³, R⁴, R⁵ und R⁶ gleich oder unterschiedlich sind und unabhängig Wasserstoff oder C₁₋₆-Alkyl sind.

2. Verwendung einer Verbindung für die Herstellung eines Medikaments zur Verwendung beim Behandeln von Schizophrenie in einem warmblütigen Lebewesen, das dieses benötigt, wobei die Verbindung die folgende Struktur (I) hat: 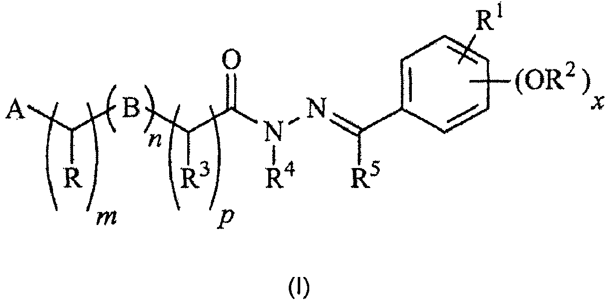 oder eines pharmazeutisch verträglichen Salzes, Stereoisomers oder Solvats davon,
wobei:
*m, n* und *p* individuell 0 oder 1 sind;
*x* 1, 2 oder 3 ist;
A ein gegebenenfalls substituierter Heterocyclus ist;
B -O-, -NR⁶- oder -S(O)_{z}- ist, wobei *z* 0, 1 oder 2 ist;
R Wasserstoff oder Oxo ist;
R¹ abwesend ist oder 1, 2 oder 3 Substituenten bedeutet, welche gleich oder unterschiedlich sind und unabhängig Halogen, C₁₋₆-Alkyl, -CHF₂, -CF₃, -CH₂NH₂, -CH₂NH(C₁₋₆-Alkyl) oder -CH₂N(C₁₋₆-Alkyl)₂ sind;
R² bei jedem Vorkommen gleich oder unterschiedlich ist und unabhängig Wasserstoff, C₁₋₆-Alkyl, -C(=O)(C₁₋₆-Alkyl), Benzyl, -CH₂CONH₂, -CHF₂, -CF₃ ist, oder beliebige zwei R²-Gruppen zusammengefasst werden können, um ein C₁₋₆-Alkandiyl zu bilden; und
R³, R⁴, R⁵ und R⁶ gleich oder unterschiedlich sind und unabhängig Wasserstoff oder C₁₋₆-Alkyl sind.

3. Verbindung zur Verwendung oder Verwendung gemäß Anspruch 1 oder 2, wobei die Verbindung die folgende Struktur (II) hat:

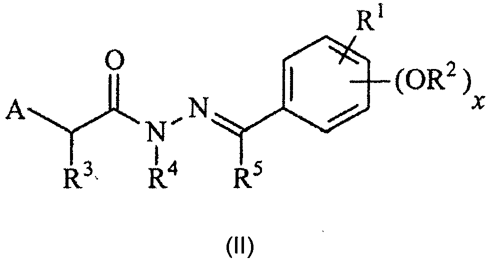

4. Verbindung zur Verwendung oder Verwendung gemäß Anspruch 1 oder 2, wobei die Verbindung die folgende Struktur (III) hat:

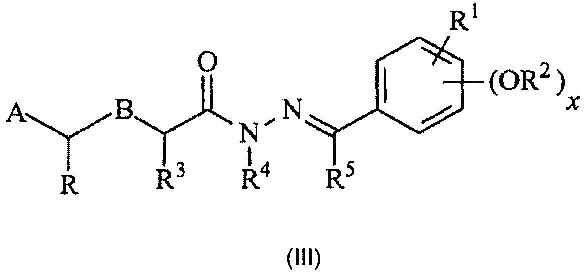

5. Verbindung zur Verwendung oder Verwendung gemäß Anspruch 1 oder 2, wobei die Verbindung die folgende Struktur (IV) hat:

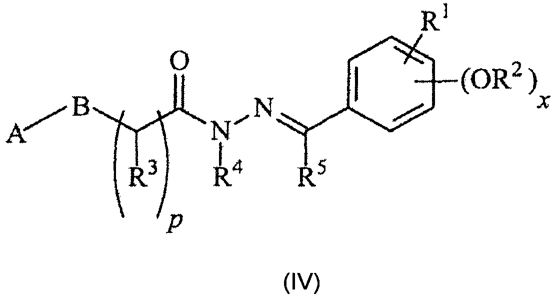

6. Verbindung zur Verwendung oder Verwendung gemäß Anspruch 1 oder 2, wobei die Verbindung die folgende Struktur (V) hat:

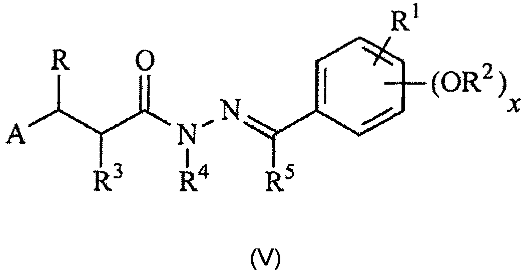

7. Verbindung zur Verwendung oder Verwendung gemäß Anspruch 1 oder 2, wobei die Verbindung die folgende Struktur (VI) hat:

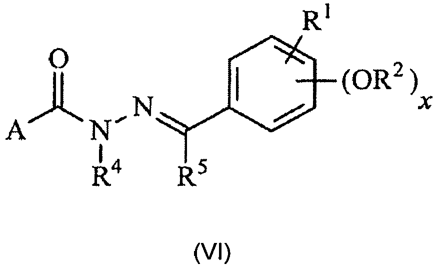

8. Verbindung zur Verwendung oder Verwendung gemäß Anspruch 1 oder 2, wobei die Verbindung die folgende Struktur (IVa) hat:

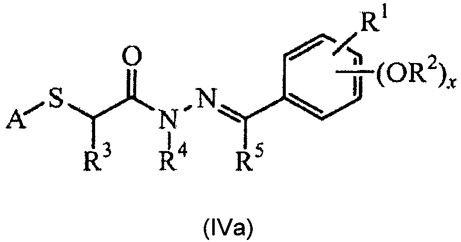

9. Verbindung zur Verwendung oder Verwendung gemäß Anspruch 1 oder 2, wobei die Verbindung die folgende Struktur (IVb) hat:

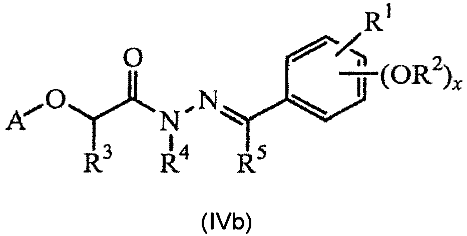

10. Verbindung zur Verwendung oder Verwendung gemäß Anspruch 1 oder 2, wobei die Verbindung die folgende Struktur (IVc) hat:

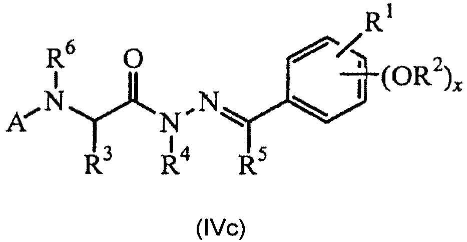

11. Verbindung zur Verwendung oder Verwendung gemäß Anspruch 1 oder 2, wobei die Verbindung die folgende Struktur (IVg) hat: 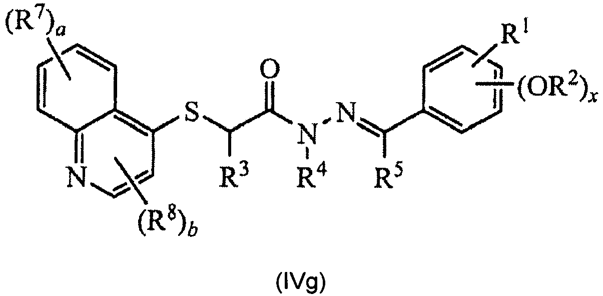 wobei:
*a* 1, 2, 3 oder 4 ist;
*b* 1 oder 2 ist; und
R⁷ und R⁸ gleich oder unterschiedlich sind und unabhängig Wasserstoff, Halogen, C₁₋₆-Alkyl, -O(C₁₋₆-Alkyl), C₁₋₆-Halogenalkyl oder Nitro sind.

12. Verbindung zur Verwendung oder Verwendung gemäß Anspruch 1 oder 2, wobei die Verbindung die folgende Struktur (IVh) hat: 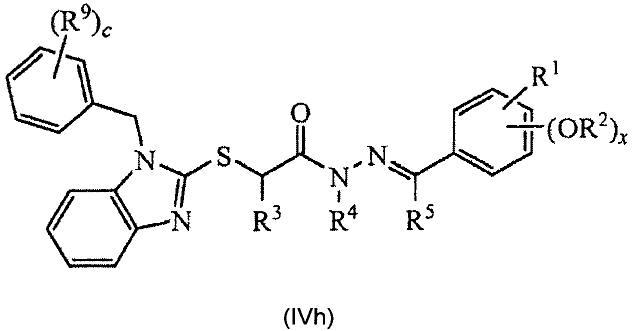 wobei:
*c* 1, 2, 3, 4 oder 5 ist; und
R⁹ bei jedem Vorkommen gleich oder unterschiedlich ist und unabhängig Wasserstoff, Halogen oder C₁₋₆-Alkyl ist.

13. Verbindung mit der folgenden Struktur (IVg): 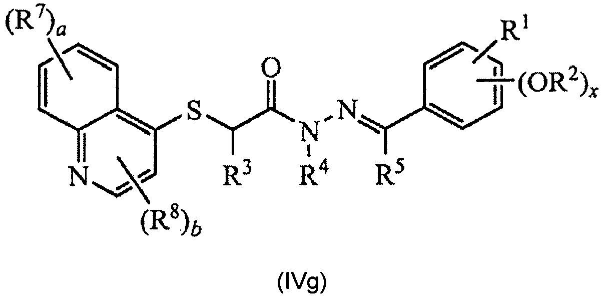 oder ein pharmazeutisch verträgliches Salz, Stereoisomer oder Solvat davon,
wobei:
*a* 1, 2, 3 oder 4 ist;
*b* 1 oder 2 ist;
*x* 1, 2 oder 3 ist, mit der Maßgabe, dass, wenn *x* 1 ist, R¹ nicht ein einzelner Methylsubstituent ist;
R¹ 1 oder 2 Substituenten bedeutet, die unabhängig Halogen, C₁₋₆-Alkyl, -CHF₂ oder -CF₃ sind;
R² bei jedem Vorkommen gleich oder unterschiedlich ist und unabhängig Wasserstoff, C₁₋₆-Alkyl, Benzyl, -CH₂CONH₂, -CHF₂, -CF₃ ist, oder beliebige zwei R²-Gruppen zusammengefasst werden können, um ein C₁₋₆-Alkandiyl zu bilden; und
R³, R⁴ und R⁵ gleich oder unterschiedlich sind und unabhängig Wasserstoff oder C₁₋₆-Alkyl sind; und
R⁷ und R⁸ gleich oder unterschiedlich sind und unabhängig Wasserstoff, Halogen, C₁₋₆-Alkyl, -O(C₁₋₆-Alkyl), C₁₋₆-Halogenalkyl oder Nitro sind.

14. Verbindung nach Anspruch 13, wobei R³, R⁴ und R⁵ Wasserstoff sind.

15. Pharmazeutische Zusammensetzung, umfassend eine Verbindung nach Anspruch 13 und einen pharmazeutisch verträglichen Träger oder ein pharmazeutisch verträgliches Verdünnungsmittel.

## Revendications

1. Composé pour l'utilisation dans le traitement de la schizophrénie chez un animal à sang chaud en ayant besoin, ledit composé ayant la structure suivante (I) : 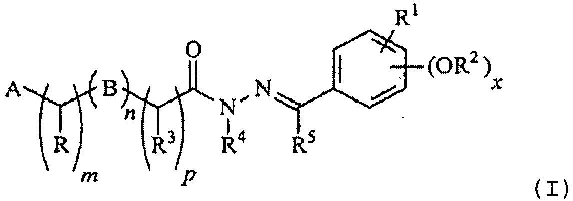 ou un sel, stéréoisomère, ou solvate pharmaceutiquement acceptable de celui-ci,
dans laquelle :
*m, n* et p ont individuellement la valeur de 0 ou 1 ;
x a la valeur de 1, 2 ou 3 ;
A est un hétérocycle éventuellement substitué ;
B est un groupe -O-, -NR⁶- ou -S(O)_{z}- où z a la valeur de 0, 1 ou 2 ;
R est un groupe hydrogéno ou oxo ;
R¹ est absent ou représente 1, 2 ou 3 substituants qui sont identiques ou différents et indépendamment un groupe halogéno, alkyle en C₁ à C₆, -CHF₂, -CF₃, -CH₂NH₂, -CH₂NH (alkyle en C₁ à C₆) ou -CH₂N(alkyle en C₁ à C₆)₂ ;
R² est à chaque apparition identique ou différent et indépendamment un groupe hydrogéno, alkyle en C₁ à C₆, -C(=O) (alkyle en C₁ à C₆), benzyle, -CH₂CONH₂, -CHF₂, -CF₃, ou deux groupes R² quelconques peuvent être pris conjointement pour former un groupe alcanediyle en C₁ à C₆ ; et
R³, R⁴, R⁵ et R⁶ sont identiques ou différents et indépendamment un groupe hydrogéno ou alkyle en C₁ à C₆.

2. Utilisation d'un composé pour la préparation d'un médicament pour l'utilisation dans le traitement de la schizophrénie chez un animal à sang chaud en ayant besoin, ledit composé ayant la structure suivante (I) : 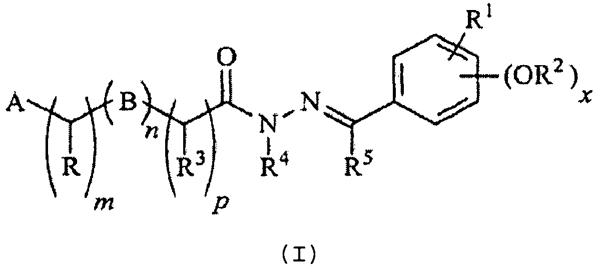 ou un sel, stéréoisomère, ou solvate pharmaceutiquement acceptable de celui-ci,
dans laquelle :
*m, n* et p ont individuellement la valeur de 0 ou 1 ;
x a la valeur de 1, 2 ou 3 ;
A est un hétérocycle éventuellement substitué ;
B est un groupe -O-, -NR⁶- ou -S(O)_{z}- où z a la valeur de 0, 1 ou 2 ;
R est un groupe hydrogéno ou oxo ;
R¹ est absent ou représente 1, 2 ou 3 substituants qui sont identiques ou différents et indépendamment un groupe halogéno, alkyle en C₁ à C₆, -CHF₂, CF₃, -CH₂NH₂, -CH₂NH(alkyle en C₁ à C₆) ou -CH₂N(alkyle en C₁ à C₆)₂ ;
R² est à chaque apparition identique ou différent et indépendamment un groupe hydrogéno, alkyle en C₁ à C₆, -C(=O) (alkyle en C₁ à C₆), benzyle, -CH₂CONH₂, -CHF₂, -CF₃, ou deux groupes R² quelconques peuvent être pris conjointement pour former un groupe alcanediyle en C₁ à C₆ ; et
R³, R⁴, R⁵ et R⁶ sont identiques ou différents et indépendamment un groupe hydrogéno ou alkyle en C₁ à C₆.

3. Composé pour l'utilisation ou utilisation selon la revendication 1 ou 2, le composé ayant la structure suivante (II) :

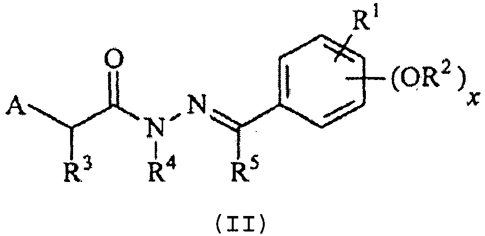

4. Composé pour l'utilisation ou utilisation selon la revendication 1 ou 2, le composé ayant la structure suivante (III) :

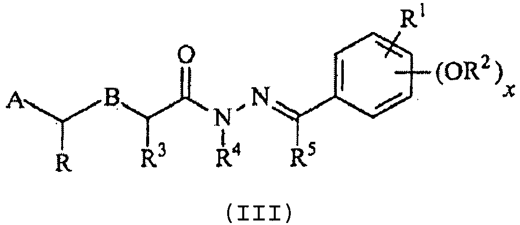

5. Composé pour l'utilisation ou utilisation selon la revendication 1 ou 2, le composé ayant la structure suivante (IV) :

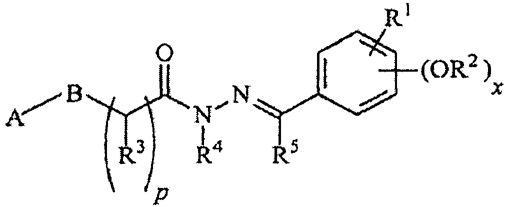

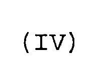

6. Composé pour l'utilisation ou utilisation selon la revendication 1 ou 2, le composé ayant la structure suivante (V) :

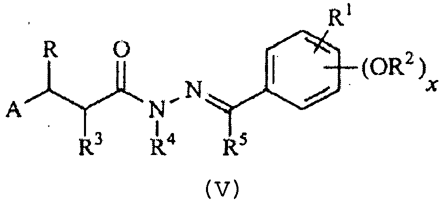

7. Composé pour l'utilisation ou utilisation selon la revendication 1 ou 2, le composé ayant la structure suivante (VI) :

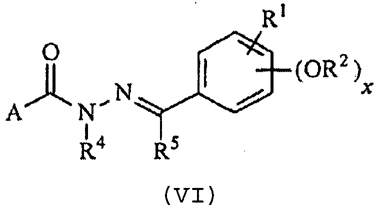

8. Composé pour l'utilisation ou utilisation selon la revendication 1 ou 2, le composé ayant la structure suivante (IVa) :

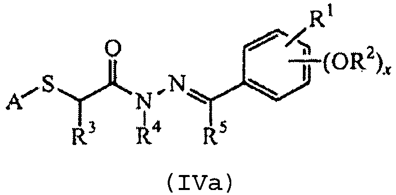

9. Composé pour l'utilisation ou utilisation selon la revendication 1 ou 2, le composé ayant la structure suivante (IVb) :

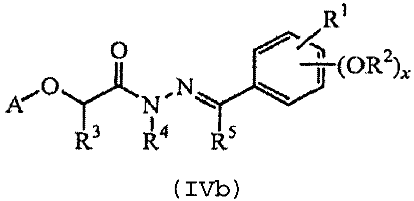

10. Composé pour l'utilisation ou utilisation selon la revendication 1 ou 2, le composé ayant la structure suivante (IVc) :

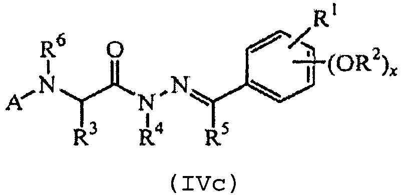

11. Composé pour l'utilisation ou utilisation selon la revendication 1 ou 2, le composé ayant la structure suivante (IVg) : 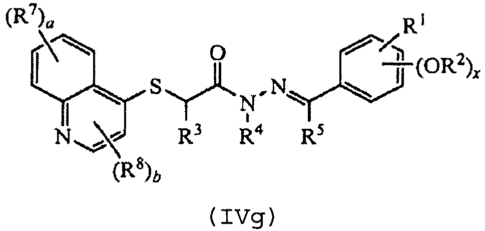 dans laquelle :
a a la valeur de 1, 2, 3 ou 4 ;
b a la valeur de 1 ou 2 ; et
R⁷ et R⁸ sont identiques ou différents et indépendamment un groupe hydrogéno, halogéno, alkyle en C₁ à C₆, -O (alkyle en C₁ à C₆), halogénoalkyle en C₁ à C₆ ou nitro.

12. Composé pour l'utilisation ou utilisation selon la revendication 1 ou 2, le composé ayant la structure suivante (IVh) : 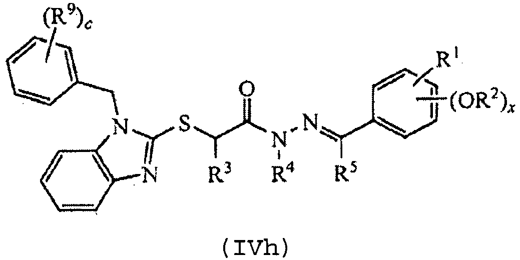 dans laquelle :
c a la valeur de 1, 2, 3, 4 ou 5 ; et
R⁹ est à chaque apparition identique ou différent et indépendamment, un groupe hydrogéno, halogéno ou alkyle en C₁ à C₆.

13. Composé ayant la structure suivante (IVg) : 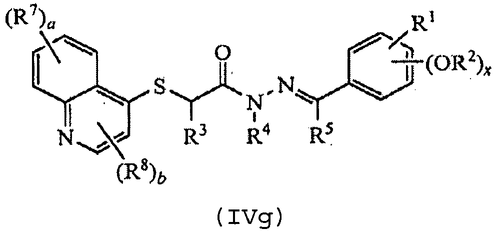 ou un sel, stéréoisomère, ou solvate pharmaceutiquement acceptable de celui-ci,
dans laquelle :
*a* a la valeur de 1, 2, 3 ou 4 ;
*b* a la valeur de 1 ou 2 ;
*x* a la valeur de 1, 2 ou 3 à condition que lorsque *x* a la valeur de 1, R¹ ne soit pas un substituant méthyle unique ;
R¹ représente 1 ou 2 substituants étant indépendamment un groupe halogéno, alkyle en C₁ à C₆,-CHF₂ ou - CF₃ ;
R² est à chaque apparition identique ou différent et indépendamment, un groupe hydrogéno, alkyle en C₁ à C₆, benzyle, -CH₂CONH₂, -CHF₂, -CF₃, ou deux groupes R² quelconques peuvent être pris conjointement pour former un groupe alcanediyle en C₁ à C₆ ; et
R³, R⁴ et R⁵ sont identiques ou différents et indépendamment un groupe hydrogéno ou alkyle en C₁ à C₆ ; et
R⁷ et R⁸ sont identiques ou différents et indépendamment un groupe hydrogéno, halogéno, alkyle en C₁ à C₆, -O(alkyle en C₁ à C₆), halogénoalkyle en C₁ à C₆ ou nitro.

14. Composé selon la revendication 13 dans lequel R³, R⁴ et R⁵ sont un groupe hydrogéno.

15. Composition pharmaceutique comprenant un composé selon la revendication 13 et un support ou un diluant pharmaceutiquement acceptable.
